(19) Europäisches Patentamt
European Patent Office
Office européen des brevets

(11) EP 2 171 028 B1

(12) **EUROPÄISCHE PATENTSCHRIFT**

(45) Veröffentlichungstag und Bekanntmachung des Hinweises auf die Patenterteilung:
**22.05.2013 Patentblatt 2013/21**

(21) Anmeldenummer: **08736071.5**

(22) Anmeldetag: **10.04.2008**

(51) Int Cl.:
***C11D 3/39*** *(2006.01)* ***C07D 471/04*** *(2006.01)*

(86) Internationale Anmeldenummer:
**PCT/EP2008/054350**

(87) Internationale Veröffentlichungsnummer:
**WO 2008/125590 (23.10.2008 Gazette 2008/43)**

(54) **BIHETEROARYL-METALLKOMPLEXE ALS BLEICHKATALYSATOREN**

BIHETEROARYL METAL COMPLEXES AS BLEACH CATALYSTS

COMPLEXES MÉTALLIQUES DE BIHÉTÉROARYLE EN TANT QUE CATALYSEURS DE BLANCHIMENT

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **12.04.2007 DE 102007017656**

(43) Veröffentlichungstag der Anmeldung:
**07.04.2010 Patentblatt 2010/14**

(73) Patentinhaber: **Henkel AG & Co. KGaA**
**40589 Düsseldorf (DE)**

(72) Erfinder:
- **HÄTZELT, Andre**
  **40591 Düsseldorf (DE)**
- **NORDSKOG, Anette**
  **3237 Sandefjord (NO)**
- **LEOPOLD, Stefan**
  **40589 Düsseldorf (DE)**
- **SCHMIEDEL, Peter**
  **40599 Düsseldorf (DE)**
- **RYBINSKI VON, Wolfgang**
  **40593 Düsseldorf (DE)**
- **SUNDERMEYER, Jörg**
  **35041 Marburg (DE)**
- **DÖRING, Jan**
  **35039 Marburg (DE)**

(56) Entgegenhaltungen:
**EP-A- 0 392 592** **WO-A-99/64554**
**DE-A1- 2 054 019** **DE-A1- 19 721 886**

- **S ROUTIER ET AL.: "Synthesis of metal complexes of 2,9-bis(2-hydroxyphenyl)-1,10-phenanthroli ne and their DNA binding and cleaving activities" JOURNAL OF THE CHEMICAL SOCIETY, PERKIN TRANS., Bd. 1998, Nr. 2, 1. Januar 1998 (1998-01-01), Seiten 863-868, XP002485253 in der Anmeldung erwähnt**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft Biheteroaryl-Metallkomplexe sowie deren Verwendung als Bleichkatalysatoren.

Zur effektiven Bleiche mit Wasserstoffperoxid muss dieses in eine bleichaktivere Spezies umgewandelt werden. Eine Möglichkeit zur Erzeugung aktivierter Peroxy-Verbindungen besteht in der Verwendung von Persäurevorläufern, sogenannter Bleichaktivatoren wie z.B. TAED, die durch Perhydrolyse in die aktive Spezies umgewandelt werden.

Eine weitere Möglichkeit zur Erzeugung aktivierter Spezies besteht in der enzymatisch katalysierten Perhydrolyse von Carbonsäureestern oder Nitrilverbindungen unter Verwendung von Perhydrolasen.

Schließlich ist es auch bekannt, Bleichkatalysatoren zur Erzeugung aktivierter Spezies zu verwenden, wobei unter einem Bleichkatalysator ein Stoff zu verstehen ist, der die Bleichleistung von Wasserstoffperoxid an einem bleichbaren Stoff verbessern kann, ohne selbst stöchiometrisch an der Reaktion beteiligt zu sein.

Die Verwendung von Bleichkatalysatoren hat gegenüber den anderen Verfahren der Bleichaktivierung den Vorteil, dass substöchiometrische Mengen der Verbindung ausreichen, wodurch in der Formulierung des bleichehaltigen Produkts eine Raum- und Gewichtsersparnis erreicht werden kann. Weiterhin ist mit der Reduzierung des Gewichts, insbesondere bei Wasch - und Reinigungsanwendungen, auch der Vorteil verknüpft, dass es zu einem geringeren Stoffeintragung in die Umwelt kommt, was aus ökologischen Gründen besonders vorteilhaft ist. Daneben können hierdurch auch Transport- und Verpackungskosten eingespart werden.

Weiterhin ist zu berücksichtigen, dass es bei Verwendung von Bleichaktivatoren wie Nitrilen oder TAED in Gegenwart von Wasser zu einer vorzeitigen Hydrolyse kommen kann, während dieses Problem bei Verwendung von Bleichkatalysatoren weitestgehend vermieden werden kann. Darüber hinaus verursacht die bei der nichtkatalytischen Bleichaktivierung ausgehend von den Persäuren erfolgende Entstehung von Säuren eine Verschiebung des pH-Wertes, die sich ungünstig auf die Bleichleistung auswirken kann. Des Weiteren ist die Bleichleistung der meisten Bleichaktivatoren bei niedrigen Temperaturen oft unzureichend.

Aus den oben genannten Gründen ist die Verwendung von Bleichkatalysatoren von besonderem Interesse gegenüber den anderen Techniken der Bleichaktivierung, so dass grundsätzlich Bedarf an neuen Bleichkatalysatoren besteht.

Als Bleichkatalysatoren sind insbesondere Metall-Komplexe von organischen Liganden wie Salenen, Saldiminen, Tris[salicylidenaminoethyl]aminen, monocyclischen Polyazaalkanen, querverbrückten polycyclischen Polyazaalkanen, Terpyridinen und Tetraamido-Liganden beschrieben. Ein Nachteil der beschriebenen Metallkomplexe besteht jedoch darin, dass sie entweder, insbesondere bei niedriger Temperatur, keine ausreichende Bleichleistung besitzen oder es aber bei ausreichender Bleichleistung zu einer unerwünschten Schädigung von Farben und gegebenenfalls auch der Textilfasern kommt.

Überraschenderweise wurde nun gefunden, dass Metallkomplexe von Biheteroaryl-Derivaten hervorragend als Bleichkatalysator geeignet sind und sich gleichzeitig schonender gegenüber Wäsche verhalten als die heute gebräuchlichen Bleichkatalysatoren.

Einige erfindungsgemäß verwendbaren Biheteroaryl-Derivate sind im Stand der Technik schon beschrieben. So beschreiben WO 00/09512 und Giblin et al. (Bioorganic & Medicinal Chemistry Letters 11 (2001) 1367-1370) 6,6'-bis-substituierte 2,2'-Bipyridine sowie auch Mangan-Komplexe dieser Verbindungen. Als Funktionen dieser Mangan-Komplexe werden Superoxid-Dismutasesowie Katalase-Aktivität und als Anwendungsmöglichkeiten die Verwendung als Antioxidantien im medizinischen Bereich genannt.

US 2003/0205707 und Lin et al. (Chem. Eur. J. 9 (2003) 1263-1272) beschreiben Platin-Komplexe, Couchma et al. (Polyhedron 18 (1999) 2633-2640) Kupfer-Komplexe und Geissman et al. (J. Org. Chem. 11 (1946) 741-750) Cobalt-Komplexe solcher Liganden. Als Verwendungsmöglichkeit für die Platin-Komplexe wird der Einsatz als Dotiersubstanz in einer Licht emittierenden Vorrichtung genannt.

Insbesondere sind einige erfindungsgemäß verwendbaren Phenantrolin-Derivate auch bereits beschrieben durch Koning et al. (ARKIVOC 2004(ii) 189-205, ISSN 1424-6376), Lam et al. (Tetrahedron 55 (1999) 8377-8384) und Routier et al. (J. Chem. Soc., Perkin Trans. 2 (1998) 863-868). Routier et al. beschreiben Kupfer-, Nickel-, Mangan- und Kobalt-Komplexe dieser Liganden sowie deren Verwendung zur Spaltung von Nukleinsäuren. Lam et al. beschreiben Kupfer- und Nickel-Komplexe und Koning et al. Kupfer- und Zink-Komplexe der jeweils beschriebenen Liganden.

Die Verwendung der Liganden und Metall-Ligand-Komplexe als Bleichkatalysatoren oder als Zusatzstoff für Wasch- und Reinigungsmittel wird in den genannten Dokumenten nicht beschrieben.

Ein erster Gegenstand der vorliegenden Erfindung sind daher Wasch- und Reinigungsmittel enthaltend substituierte Biheteroaryl-Liganden sowie Biheteroaryl-Ligand-Komplexe von Biheteroaryl-Liganden der allgemeinen Formel (I)

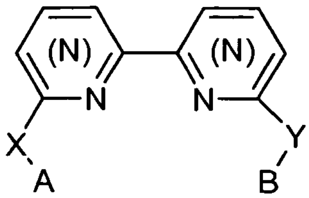

(I),

wobei

X und Y unabhängig voneinander für Ethyl, Ethenyl, -CH=N-, -C($C_{1-18}$-Alkyl)=N-, Cycloalkyl, Cycloheteroalkyl, $C_{6-10}$-Aryl oder Heteroaryl stehen,

A und B unabhängig voneinander für OH, SH, $NH_2$, NH($C_{1-18}$-Alkyl) oder N($C_{1-18}$-Alkyl)$_2$ stehen, wobei A durch X und B durch Y über 2 Atome mit dem Biheteroaryl-Rest verknüpft sind,

und wobei das so gebildete Gerüst auch verbrückt sowie durch weitere Substituenten ein- oder mehrfach substituiert sein kann,

und wobei (N) bedeutet, dass optional ein oder zwei CH-Gruppen des entsprechenden Arylrests durch N ersetzt sein können.

Bei dem substituierten Biheteroaryl-Liganden kann es sich insbesondere um einen Liganden ausgewählt aus den folgenden Liganden handeln:

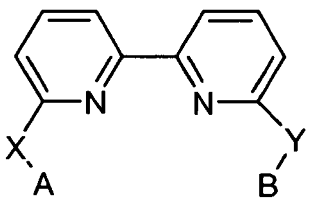
,
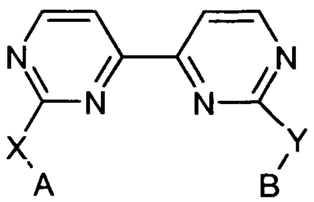
,
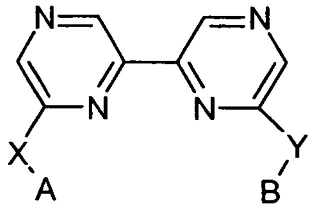
,

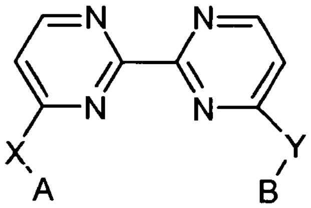
,
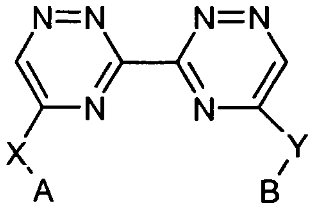
,
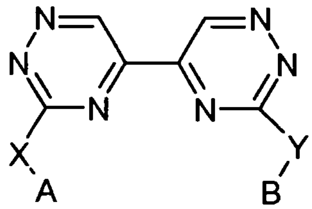
.

wobei der Ligand jeweils entsprechend verbrückt sowie durch weitere Substituenten ein- oder mehrfach substituiert sein kann.

Als Beispiele seien insbesondere genannt die Liganden baobipy und bfobipy:

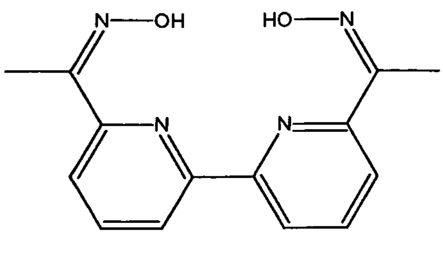

baobipy

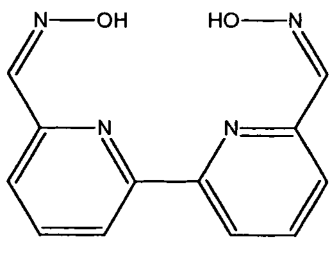

bfobipy

Bei dem verbrückten Biheteroaryl-Liganden handelt es sich vorzugsweise um einen Liganden der allgemeinen Formel (II)

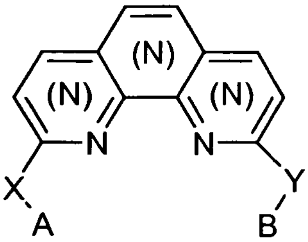

(II),

wobei das so gebildete Gerüst auch ein- oder mehrfach substituiert sein kann.

Der verbrückte Biheteroaryl-Ligand kann insbesondere aus folgender Gruppe ausgewählt sein:

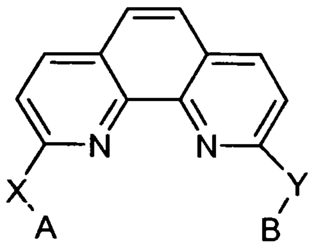

,

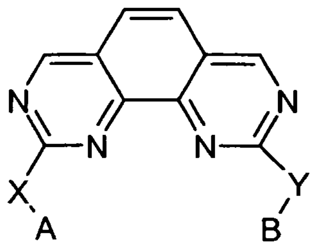

,

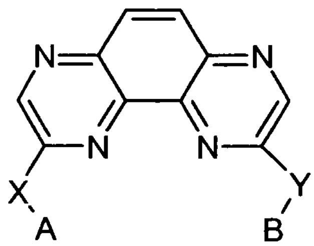

,

wobei das so gebildete Gerüst auch ein- oder mehrfach substituiert sein kann.

Als Beispiele genannt seien insbesondere die Liganden baophen und bfophen:

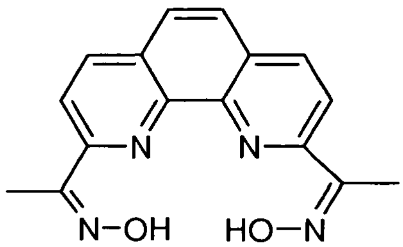

baophen

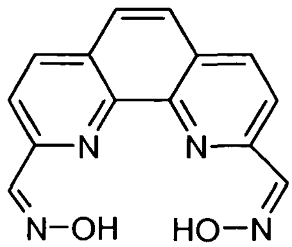

bfophen

In einer bevorzugten Ausführungsform stehen X und Y unabhängig voneinander für $C_{6-10}$-Aryl oder Heteroaryl und A und B für OH oder SH.

X und Y sind vorzugsweise ausgewählt aus gegebenenfalls substituiertem Benzol, Naphthalin, Pyrrol, Furan, Thiophen, Pyridin, Pyrimidin, Pyrazin, Pyron, Pyridon, Purin, Triazin, Imidazol, Thiazol, Oxazol, Indol, Chinolin, Isochinolin, Benzimidazol, Benzthiazol und Benzoxazol.

In einer weiteren bevorzugten Ausführungsform handelt es sich bei den erfindungsgemäßen Biheteroaryl-Liganden um Liganden der allgemeinen Formel (III)

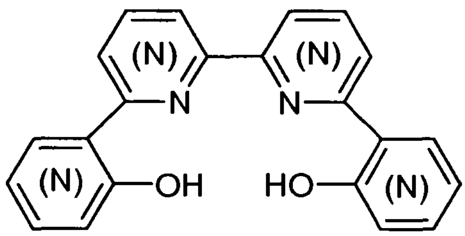

(III)

wobei das so gebildete Gerüst auch verbrückt sowie ein- oder mehrfach substituiert sein kann.

Insbesondere kann es sich hierbei bei den verbrückten Biheteroaryl-Liganden um Liganden der allgemeinen Formel (IV)

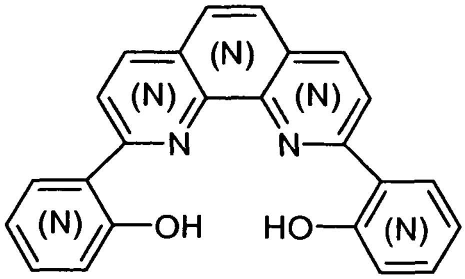

(IV)

handeln,
wobei das so gebildete Gerüst auch ein- oder mehrfach substituiert sein kann.

In einer besonders bevorzugten Ausführungsform handelt es sich bei dem substituierten Biheteroaryl-Liganden um einen substituierten Phenantrolin-Liganden der allgemeinen Formel (V)

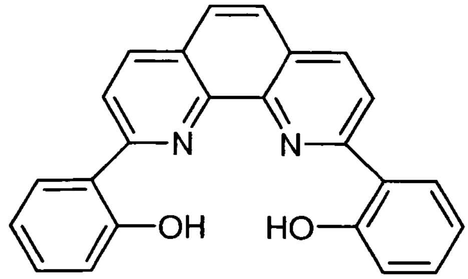

(V)

wobei das so gebildete Gerüst auch ein- oder mehrfach substituiert sein kann.

Die Substituenten, die an die zuvor genannten Grundstrukturen gebunden sein können, können insbesondere ausgewählt sein aus Alkyl, insbesondere $C_{1-22}$-Alkyl, vorzugsweise $C_{1-18}$-Alkyl, Trifluormethyl, Cycloalkyl, insbesondere $C_{3-8}$-Cycloalkyl, Cycloalkylalkyl, insbesondere $C_{3-8}$-Cycloalkyl-$C_{1-12}$-alkyl, Alkenyl, insbesondere $C_{2-18}$-Alkenyl, Alkinyl, insbesondere $C_{2-18}$-Alkinyl, Heteroalkyl, Heterocycloalkyl, Alkoxy, insbesondere $C_{1-18}$-Alkoxy, Alkylsulfanyl, insbesondere $C_{1-18}$-Alkylsulfanyl, Alkylsulfinyl, insbesondere $C_{1-18}$-Alkylsufinyl, Alkylsulfonyl, insbesondere $C_{1-18}$-Alkylsulfonyl, Alkanoyl, insbesondere $C_{1-18}$-Alkanoyl, Alkanoyloxy, insbesondere $C_{1-18}$-Alkanoyloxy, Alkoxycarbonyl, insbesondere $C_{1-18}$-Alkoxycarbonyl, Alkylaminocarbonyl, insbesondere $C_{1-18}$-Alkylaminocarbonyl, Alkylsulfanylcarbonyl, insbesondere $C_{1-18}$-Alkylsulfanylcarbonyl, Hydroxy, Amino, Aryl, insbesondere $C_{6-10}$-Aryl, Arylalkyl, insbesondere $C_{6-10}$-Aryl-$C_{1-12}$-alkyl, Aryloxy, insbesondere $C_{6-10}$-Aryloxy, Arylsulfanyl, insbesondere $C_{6-10}$-Arylsulfanyl, Arylsulfinyl, insbesondere $C_{6-10}$-Arylsulfinyl, Arylsulfonyl, insbesondere $C_{6-10}$-Arylsulfonyl, Arylcarbonyl, insbesondere $C_{6-10}$-Arylcarbonyl, Arylcarbonyloxy, insbesondere $C_{6-10}$-Arylcarbonyloxy, Aryloxycarbonyl, insbesondere $C_{6-10}$-Aryloxycarbonyl, Arylaminocarbonyl, insbesondere $C_{6-10}$-Arylaminocarbonyl, Arylsulfanylcarbonyl, insbesondere $C_{6-10}$-Arylsulfanylcarbonyl, Heteroaryl, Heteroarylalkyl, insbesondere Heteroaryl-$C_{1-12}$-alkyl, Heteroaryloxy, Heteroarylamino, Heteroarylsulfanyl, Heteroarylsulfonyl, Heteroarylsulfoxidyl, Heteroarylcarbonyl, Heteroarylcarbonyloxy, Heteroaryloxycarbonyl, Heteroarylaminocarbonyl, Heteroarylsulfanylcarbonyl, Alkoxysulfonyl, insbesondere $C_{1-18}$-Alkoxysulfonyl, Alkoxycarbinol, insbesondere $C_{1-12}$-Alkoxycarbinol, Ammonium, Hydroxycarbonyl, Alkoxycarbonyl, insbesondere $C_{1-18}$-Alkoxycarbonyl, Aryloxycarbonyl, insbesondere $C_{6-10}$-Aryloxycarbonyl, Amidocarbonyl, Halogen, insbesondere Chlor, Brom, Iod oder Fluor, Nitro, Sulfato, Sulfo, Amidosulfo, Phosphato, Phosphono, Amidophosphono, Formyl, Thioformyl, $-(CH_2-CH_2-O-)_nH$ und $-(CH_2-CH_2-CH_2-O)_nH$ mit n = 1 bis 20, vorzugsweise 3 bis 20, wobei alle Reste des sich so ergebenden Moleküls, insbesondere die aliphatischen und aromatischen Reste jeweils unabhängig voneinander gegebenenfalls auch ein- oder mehrfach, insbesondere ein-, zwei- oder dreifach, vorzugsweise einfach, substituiert sein können, insbesondere durch Substituenten ausgewählt aus den zuvor genannten Resten.

Die Substituenten, die an das Grundgerüst gebunden sein können, sind vorzugsweise ausgewählt aus Alkyl, insbesondere $C_{1-22}$-Alkyl, vorzugsweise $C_{1-18}$-Alkyl, Cycloalkyl, insbesondere $C_{3-8}$-Cycloalkyl, Cycloalkylalkyl, insbesondere $C_{3-8}$-Cycloalkyl-$C_{1-12}$-alkyl, Alkenyl, insbesondere $C_{2-18}$-Alkenyl, Alkinyl, insbesondere $C_{2-18}$-Alkinyl, Heteroalkyl, Heterocycloalkyl, Alkoxy, insbesondere $C_{1-18}$-Alkoxy, Alkanoyl, insbesondere $C_{1-18}$-Alkanoyl, Alkoxycarbonyl, insbesondere $C_{1-18}$-Alkoxycarbonyl, Alkylaminocarbonyl, insbesondere $C_{1-18}$-Alkylaminocarbonyl, Alkylsulfanylcarbonyl, insbesondere $C_{1-18}$-Alkylsulfanylcarbonyl, Hydroxy, Amino, Alkylamino, insbesondere ($C_{1-18}$-Alkyl)NH oder Di-($C_{1-18}$-Alkyl)N, Aryl, insbesondere $C_{6-10}$-Aryl, Arylalkyl, insbesondere $C_{6-10}$-Aryl-$C_{1-12}$-alkyl, Arylcarbonyl, insbesondere $C_{6-10}$-Arylcarbonyl, Aryloxycarbonyl, insbesondere $C_{6-10}$-Aryloxycarbonyl, Arylaminocarbonyl, insbesondere $C_{6-10}$-Aryl-

aminocarbonyl, Arylsulfanylcarbonyl, insbesondere $C_{6-10}$-Arylsulfanylcarbonyl, Heteroaryl, Heteroarylalkyl, insbesondere Heteroaryl-$C_{1-12}$-alkyl, Heteroarylcarbonyl, Heteroaryloxycarbonyl, Heteroarylaminocarbonyl, Heteroarylsulfanylcarbonyl, Trifluormethyl, Ammonium, Hydroxycarbonyl, Alkoxycarbonyl, insbesondere $C_{1-18}$-Alkoxycarbonyl, Aryloxycarbonyl, insbesondere $C_{6-10}$-Aryloxycarbonyl, Amidocarbonyl, Halogen, insbesondere Chlor, Brom, lod oder Fluor, Nitro, Sulfato, Sulfo, Amidosulfo, Phosphato, Phosphono, Amidophosphono, Formyl, Thioformyl, $-(CH_2-CH_2-O-)_nH$ und $-(CH_2-CH_2-CH_2-O)_nH$ mit n = 1 bis 20, vorzugsweise 3 bis 20,
wobei alle Reste des sich so ergebenden Moleküls, insbesondere die aliphatischen und aromatischen Reste, jeweils unabhängig voneinander gegebenenfalls auch ein- oder mehrfach, insbesondere ein-, zwei- oder dreifach, vorzugsweise einfach, substituiert sein können, insbesondere durch Substituenten ausgewählt aus den zuvor genannten Resten.

Bei den Substituenten, die an das Grundgerüst gebunden sein können, handelt es sich in einer besonders bevorzugten Ausführungsform um Substituenten, die die Löslichkeit des Liganden in wässrigem Medium erhöhen. An die erfindungsgemäßen Liganden ist daher in einer besonders bevorzugten Ausführungsform mindestens ein Substituent umfassend einen Ammonium-, Hydroxycarbonyl-, Alkoxycarbonyl-, Aryloxycarbonyl-, Amidocarbonyl-, Halogen-, Nitro-, Sulfato-, Sulfo-, Amidosulfo-, Phosphato-, Phosphono-, Amidophosphono-, Hydroxy-, Alkoxy-, Amino- oder Polyoxyethylen-Rest gebunden. Bei dem Substituenten kann es sich insbesondere um den jeweiligen Rest selbst handeln oder um eine Alkylgruppe, an die der jeweilige Rest gebunden ist, so dass der Substituent besonders bevorzugt ausgewählt ist aus Sulfo, Sulfoalkyl, insbesondere Sulfo-$C_{1-18}$-Alkyl, Hydroxycarbonyl, Hydroxycarbonylalkyl, insbesondere Hydroxycarbonyl-$C_{1-18}$-alkyl, Phosphono, Phosphonoalkyl, insbesondere Phosphono-$C_{1-18}$-alkyl Hydroxy, Hydroxyalkyl, insbesondere Hydroxy-$C_{1-18}$-alkyl, Amino, Aminoalkyl, insbesondere Amino-$C_{1-18}$-alkyl, Halogen, Haloalkyl, insbesondere Halo-$C_{1-18}$-alkyl, $-(CH_2-CH_2-O-)_nH$ und $C_{1-18}$-alkyl-$(CH_2-CH_2-O-)_nH$ mit jeweils n = 1 bis 20, vorzugsweise 3 bis 20.

$C_{1-18}$-Alkyl steht erfindungsgemäß jeweils unabhängig voneinander für alle gesättigten linearen und verzweigten Alkyl-Reste mit bis zu 18 C-Atomen, wobei $C_{1-6}$-Alkyl-Reste bevorzugt sind. $C_{1-6}$-Alkyl steht erfindungsgemäß für alle gesättigten linearen und verzweigten Alkyl-Reste mit bis zu 6 C-Atomen, insbesondere für Methyl, Ethyl, n-Propyl, i-Propyl, n-Butyl, i-Butyl, t-Butyl sowie alle Isomere des Pentyl und des Hexyl.

$C_{3-8}$-Cycloalkyl steht erfindungsgemäß jeweils unabhängig voneinander für alle cyclischen Alkyl-Reste mit 3 bis 8 C-Atomen, vorzugsweise mit 5 bis 6 C-Atomen, wobei die Reste gesättigt oder ungesättigt sein können, insbesondere für Cyclopentyl, Cyclohexyl oder Cyclopentadienyl.

$C_{2-18}$-Alkenyl steht erfindungsgemäß jeweils unabhängig voneinander für alle linearen und verzweigten Alkyl-Reste mit bis zu 18 C-Atomen, die mindestens eine Doppelbindung enthalten, wobei $C_{2-6}$-Alkenyl-Reste bevorzugt sind. $C_{2-6}$-Alkenyl steht erfindungsgemäß für alle linearen und verzweigten Alkyl-Reste mit bis zu 6 C-Atomen, die mindestens eine Doppelbindung enthalten, insbesondere für Ethenyl, Propenyl, i-Propenyl sowie alle Isomere des Butenyl, Pentenyl und Hexenyl.

$C_{2-18}$-Alkinyl steht erfindungsgemäß jeweils unabhängig voneinander für alle linearen und unverzweigten Alkyl-Reste mit bis zu 18 C-Atomen, die mindestens eine Dreifachbindung enthalten, wobei $C_{2-6}$-Alkinyl-Reste bevorzugt sind. $C_{2-6}$-Alkinyl steht erfindungsgemäß für alle linearen und unverzweigten Alkyl-Reste mit bis zu 6 C-Atomen, die mindestens eine Dreifachbindung enthalten, insbesondere für Ethinyl, Propinyl, i-Propinyl sowie alle Isomere des Butinyl, Pentinyl und Hexinyl.

Heteroalkyl steht erfindungsgemäß jeweils unabhängig voneinander für alle gesättigten und ein - oder mehrfach ungesättigten, linearen oder verzweigten Alkyl-Reste, die mindestens ein, bevorzugt genau ein Heteroatom, insbesondere ausgewählt aus O, S und N, enthalten, wobei die Summe aus C- und Hetero-Atomen bevorzugt bis zu 18, besonders bevorzugt bis zu 6, beträgt.

Heterocycloalkyl steht erfindungsgemäß jeweils unabhängig voneinander für alle cyclischen Alkyl-Reste, die mindestens ein, bevorzugt genau ein, Heteroatom, insbesondere ausgewählt aus O, S oder N, enthalten, wobei der Ring vorzugsweise drei- bis achtgliederig, besonders bevorzugt fünfbis sechsgliedrig ist. Beispiele hierfür sind Tetrahydrofuranyl, Tetrahydrothiophenyl, Pyrrolidinyl, 2-Thiazolinyl, Tetrahydrothiazolyl, Tetrahydrooxazolyl, Piperidinyl, Piperazinyl, Morpholinyl und Thiomorpholinyl.

$C_{1-18}$-Alkoxy steht erfindungsgemäß jeweils unabhängig voneinander für alle gesättigten und ungesättigten, linearen und verzweigten Alkyl-Reste mit bis zu 18 C-Atomen, die über ein Sauerstoff-Atom gebunden sind, wobei $C_{1-6}$-Alkoxy-Reste bevorzugt sind. $C_{1-6}$-Alkoxy steht erfindungsgemäß jeweils unabhängig voneinander für alle gesättigten und ungesättigten, linearen und verzweigten Alkyl-Reste mit bis zu 6 C-Atomen, die über ein Sauerstoff-Atom gebunden sind, insbesondere für Methoxy und Ethoxy.

$C_{1-18}$-Alkylsulfanyl steht erfindungsgemäß jeweils unabhängig voneinander für alle gesättigten und ungesättigten, linearen und verzweigten Alkyl-Reste mit bis zu 18 C-Atomen, die über ein Schwefel-Atom gebunden sind, wobei $C_{1-6}$-Alkylsulfanyl-Reste bevorzugt sind. $C_{1-6}$-Alkylsulfanyl steht erfindungsgemäß für alle gesättigten und ungesättigten, linearen und verzweigten Alkyl-Reste mit bis zu 6 C-Atomen, die über ein Schwefel-Atom gebunden sind, insbesondere für Methysulfanyl und Ethylsulfanyl.

$C_{1-18}$-Alkylsulfinyl steht erfindungsgemäß jeweils unabhängig voneinander für alle gesättigten und ungesättigten, linearen und verzweigten Alkyl-Reste mit bis zu 18 C-Atomen, die über eine SO-Gruppe gebunden sind, wobei

$C_{1-6}$-Alkylsulfonyl-Reste bevorzugt sind. $C_{1-6}$-Alkylsulfinyl steht erfindungsgemäß für alle gesättigten und ungesättigten, linearen und verzweigten Alkyl-Reste mit bis zu 6 C-Atomen, die über eine SO-Gruppe gebunden sind, insbesondere für Methylsulfinyl und Ethylsulfinyl.

$C_{1-18}$-Alkylsulfonyl steht erfindungsgemäß jeweils unabhängig voneinander für alle gesättigten und ungesättigten, linearen und verzweigten Alkyl-Reste mit bis zu 18 C-Atomen, die über eine $SO_2$-Gruppe gebunden sind, wobei $C_{1-8}$-Alkylsulfoxidyl-Reste bevorzugt sind. $C_{1-6}$-Alkylsulfonyl steht erfindungsgemäß für alle gesättigten und ungesättigten, linearen und verzweigten Alkyl-Reste mit bis zu 6 C-Atomen, die über eine $SO_2$-Gruppe gebunden sind, insbesondere für Methylsulfonyl und Ethylsutfonyl.

$C_{1-18}$-Alkanoyl steht erfindungsgemäß jeweils unabhängig voneinander für alle gesättigten und ungesättigten, linearen und verzweigten Alkyl-Reste mit bis zu 18 C-Atomen, die über eine Carbonyl-Gruppe gebunden sind, wobei $C_{1-6}$-Alkanoyl-Reste bevorzugt sind. $C_{1-8}$-Alkanoyl steht erfindungsgemäß für alle gesättigten und ungesättigten, linearen und verzweigten Alkyl-Reste mit bis zu 6 C-Atomen, die über eine Carbonyl-Gruppe gebunden sind, insbesondere für Methycarbonyl und Ethylcarbonyl.

$C_{1-18}$-Alkanoyloxy steht erfindungsgemäß jeweils unabhängig voneinander für alle gesättigten und ungesättigten, linearen und verzweigten Alkyl-Reste mit bis zu 18 C-Atomen, die über eine Carbonyloxy-Gruppe gebunden sind, wobei $C_{1-6}$-Alkanoyloxy-Reste bevorzugt sind. $C_{1-6}$-Alkanoyloxy steht erfindungsgemäß für alle gesättigten und ungesättigten, linearen und verzweigten Alkyl-Reste mit bis zu 6 C-Atomen, die über eine Carbonyloxy-Gruppe gebunden sind, insbesondere für Methanoyloxy, Ethanoyloxy, n-Propanoyloxy und i-Propanoyloxy.

$C_{1-18}$-Alkoxycarbonyl steht erfindungsgemäß jeweils unabhängig voneinander für alle gesättigten und ungesättigten, linearen und verzweigten Alkyl-Reste mit bis zu 18 C-Atomen, die über eine Oxycarbonyl-Gruppe gebunden sind, wobei $C_{1-6}$-Alkoxycarbonyl-Reste bevorzugt sind. $C_{1-6}$-Alkoxycarbonyl steht erfindungsgemäß für alle gesättigten und ungesättigten, linearen und verzweigten Alkyl-Reste mit bis zu 6 C-Atomen, die über eine Oxycarbonyl-Gruppe gebunden sind, insbesondere für Methoxycarbonyl und Ethoxycarbonyl.

$C_{1-18}$-Alkylaminocarbonyl steht erfindungsgemäß jeweils unabhängig voneinander für eine Aminocarbonyl-Gruppe, die ein- oder zweifach durch einen gesättigten oder ungesättigten, linearen oder verzweigten Alkyl-Rest mit bis zu 18 C-Atomen substituiert ist, wobei ein- oder zweifach durch $C_{1-6}$-Alkyl-Gruppen substituierte Aminocarbonyl-Reste, insbesondere Monomethylaminocarbonyl, Diemethylaminocarbonyl, Monoethylaminocarbonyl und Diethylaminocarbonyl, bevorzugt sind.

$C_{1-18}$-Alkylsulfanylcarbonyl steht erfindungsgemäß jeweils unabhängig voneinander für alle gesättigten und ungesättigten, linearen und verzweigten Alkyl-Reste mit bis zu 18 C-Atomen, die über eine Thiocarbonyl-Gruppe gebunden sind, wobei $C_{1-6}$-Alkylsulfanylcarbonyl-Reste bevorzugt sind. $C_{1-6}$-Alkylsulfanylcarbonyl steht erfindungsgemäß für alle gesättigten und ungesättigten, linearen und verzweigten Alkyl-Reste mit bis zu 6 C-Atomen, die über eine Thiocarbonyl-Gruppe gebunden sind, insbesondere für Methylthiocarbonyl und Ethylthiocarbonyl.

($C_{1-18}$-Alkyl)NH steht erfindungsgemäß jeweils unabhängig voneinander für alle gesättigten und ungesättigten, linearen und verzweigten Alkylreste mit bis zu 18 C-Atomen, die über eine Hydrogenamino-Gruppe gebunden sind, wobei ($C_{1-6}$-Alkyl)NH bevorzugt ist. ($C_{1-6}$-Alkyl)NH steht erfindungsgemäß für alle gesättigten und ungesättigten, linearen und verzweigten Alkyl-Reste mit bis zu 6 C-Atomen, die über eine Hydrogenamino-Gruppe gebunden sind, insbesondere für $CH_3NH$ und $C_2H_5NH$.

Di-($C_{1-18}$-Alkyl)N steht erfindungsgemäß jeweils unabhängig voneinander für alle gesättigten und ungesättigten, linearen und verzweigten Alkylreste mit bis zu 18 C-Atomen, die über eine ($C_{1-18}$-Alkyl)amino-Gruppe gebunden sind, wobei Di-($C_{1-6}$-Alkyl)N bevorzugt ist. Die beiden Alkyl-Reste können hierbei gleich oder unterschiedlich voneinander sein. Di-($C_{1-6}$-Alkyl)N steht erfindungsgemäß für alle gesättigten und ungesättigten, linearen und verzweigten Alkylreste mit bis zu 6 C-Atomen, die über eine ($C_{1-6}$-Alkyl)amino-Gruppe gebunden sind, insbesondere für $(CH_3)_2N$ und $(C_2H_5)_2N$.

$C_{6-10}$-Aryl steht erfindungsgemäß, insbesondere auch in $C_{6-10}$-Aryl-$C_{1-12}$-alkyl, $C_{6-10}$-Aryloxy, $C_{6-10}$-Arylamino, $C_{6-10}$-Arylsulfanyl, $C_{6-10}$-Arylsulfonyl, $C_{6-10}$-Arylsulfoxidyl, $C_{6-10}$-Arylcarbonyl, $C_{6-10}$-Arylcarbonyloxy, $C_{6-10}$-Aryloxycarbonyl, $C_{6-10}$-Arylaminocarbonyl und $C_{6-10}$-Arylsulfanylcarbonyl, vorzugsweise für Phenyl oder Naphthyl, besonders bevorzugt für Phenyl.

Heteroaryl steht erfindungsgemäß, insbesondere auch in Heteroaryl-$C_{1-12}$-alkyl, Heteroaryloxy, Heteroarylamino, Heteroarylsulfanyl, Heteroarylsulfonyl, Heteroarylsulfoxidyl, Heteroarylcarbonyl, Heteroarylcarbonyloxy, Heteroaryloxycarbonyl, Heteroarylaminocarbonyl und Heteroarylsulfanylcarbonyl, sofern nicht anders angegeben, für einen mindestens ein Heteroatom ausgewählt aus O, S und N enthaltenden aromatischen Rest mit 5 bis 10, vorzugsweise 5 oder 6, Ringgliedern, vorzugsweise ausgewählt aus Furanyl, Thienyl, Thiophenyl, Pyrrolyl, Isopyrrolyl, Pyrazolyl, Imidazolyl, Oxazolyl, Thiazolyl, Isothiazolyl, Pyridinyl, Pyridazinyl, Pyrimidinyl, Pyrazinyl, Triazinyl, Benzofuranyl, Benzothiophenyl, Indolyl, Chinolinyl, Isochinolinyl, Benzimidazolyl, Indazolyl, Pyridofuranyl und Pyridothienyl.

In $C_{6-10}$-Aryl-$C_{1-12}$-alkyl und Heteroarylalkyl kann der Alkyl-Rest gesättigt oder ungesättigt, verzweigt oder unverzweigt sein. Bevorzugte Reste sind Benzyl, Phenylethyl, Naphthylmethyl und Naphthylethyl.

"Amino" steht erfindungsgemäß für jede beliebige substituierte oder unsubstituierte Aminogruppe, insbesondere für $-NH_2$, $-NH(C_{1-18}\text{-Alkyl})$, $-N(C_{1-18}\text{-Alkyl})_2$, $-NH(C_{6-10}\text{-Aryl})$ oder $-N(C_{6-10}\text{-Aryl})_2$.

"Ammonium" steht erfindungsgemäß für jede beliebige substituierte oder unsubstituierte Ammoniumgruppe, insbesondere für $-NH_3^{(+)}$, $-NH_2(C_{1-18}\text{-Alkyl})^{(+)}$, $-NH(C_{1-18}\text{-Alkyl})_2^{(+)}$ oder $-N(C_{1-18}\text{-Alkyl})_3^{(+)}$,

"Sulfato" steht erfindungsgemäß insbesondere für $-O-S(O)_2-O-R$, "Sulfo" für $-S(O)_2-O-R$, "Amidosulfo" für $-O-S(O)_2-NR_2$, "Phosphato" für $-O-P(O)(OR)_2$, "Phosphono" für $-P(O)(OR)_2$ "Amidophosphono" für $-O-P(O)(NR_2)_2$ oder $-O-P(O)(OR)(NR_2)$ und "Amidocarbonyl" für $-C(O)-NR_2$, wobei R jeweils unabhängig voneinander für H, $M^{(+)}$, $C_{1-18}$-Alkyl, $C_{6-10}$-Aryl oder $C_{1-18}$-Alkyl-$G_{6-10}$-Aryl steht.

Bei dem erfindungsgemäßen Metall-Ligand-Komplex handelt es sich vorzugsweise um einen Komplex mit einem Metall ausgewählt aus Ag, Al, Ce, Co, Cu, Fe, Mo, Mn, Ni, Pb, Re, Ti, V und Zn in beliebigen Oxidationsstufen, wobei das Metall vorzugsweise ausgewählt ist aus Co(II), Co(III), Cu(I), Cu(II), Fe(II), Fe(III), Mn(II), Mn(III), Ni(II), Pb (II) und Zn(II), besonders bevorzugt aus Mn(II) und Mn(III).

Die Herstellung des Metall-Ligand-Komplexes kann in der Regel auf einfache Weise dadurch erfolgen, dass ein Metallsalz des entsprechenden Metalls mit dem entsprechenden Ligand in wässriger Umgebung vermischt wird. Durch Einstellen eines geeigneten Redoxpotentials kann die Entstehung einer gewünschten Oxidationsstufe begünstigt werden.

Zur Absättigung der nach Bindung an den Liganden noch freien Valenzen und/oder noch freien Ladung kommt grundsätzlich jedes beliebige Gegenion in Frage, insbesondere Acetat, Tetrafluoroborat, Fluorid, Bromid, Iodid oder Chlorid.

Weiterer Gegenstand der vorliegenden Erfindung ist ebenfalls die Verwendung erfindungsgemäßer Wasch- und Reinigungsmittel zur Reinigung textiler Flächengebilde sowie zur Reinigung harter Oberflächen.

Gegenstand der vorliegenden Erfindung sind ebenfalls die zuvor genannten erfindungsgemäßen Liganden sowie Metall-Ligand-Komplexe als solche. Die erfindungsgemäßen Metall-Ligand-Komplexe werden im Folgenden auch "erfindungsgemäße Bleichkatalysatoren" genannt.

Ein besonderer Gegenstand der vorliegenden Erfindung sind Metall-Ligand-Komplexe von Biheteroaryl-Liganden der allgemeinen Formel (IV), vorzugsweise von Biheteroaryl-Liganden der allgemeinen Formel (V),
dadurch gekennzeichnet, dass das Übergangsmetall ausgewählt ist aus Ag, Al, Ce, Co, Cu, Fe, Mo, Mn, Pb, Re, Ti, V und Zn in beliebigen Oxidationsstufen, insbesondere aus Co(II), Co(III), Cu(I), Cu(II), Fe(II), Fe(III), Mn(II), Mn(III), Pb (II) und Zn(II), besonders bevorzugt aus Mn(II) und Mn(III),
und dass mindestens ein Substituent umfassend einen Ammonium-, Hydroxycarbonyl-, Alkoxycarbonyl-, Aryloxycarbonyl-, Amidocarbonyl-, Halogen-, Nitro-, Sulfato-, Sulfo-, Amidosulfo-, Phosphato-, Phosphono-, Amidophosphono-, Hydroxy-, Alkoxy-, Amino- oder Polyoxyethylen-Rest, insbesondere $-(CH_2-CH_2-O-)_n-$ mit n = 1 bis 20, vorzugsweise 3 bis 20, an das Liganden-Gerüst gebunden ist, wobei es sich in einer bevorzugten Ausführungsform bei dem Substituenten insbesondere um den jeweiligen Rest selbst handeln kann oder um eine Alkylgruppe, an die der jeweilige Rest gebunden ist, so dass der Substituent bevorzugt ausgewählt ist aus Sulfo, Sulfoalkyl, insbesondere Sulfo-$C_{1-18}$-Alkyl, Hydroxycarbonyl, Hydroxycarbonylalkyl, insbesondere Hydroxycarbonyl-$C_{1-18}$-alkyl, Phosphono, Phosphonoalkyl, insbesondere Phosphono-$C_{1-18}$-alkyl Hydroxy, Hydroxyalkyl, insbesondere Hydroxy-$C_{1-18}$-alkyl, Amino, Aminoalkyl, insbesondere Amino-$C_{1-18}$-alkyl, Halogen, Haloalkyl, insbesondere Halo-$C_{1-18}$-alkyl, $-(CH_2-CH_2-O-)_nH$ und $C_{1-18}$-alkyl-$(CH_2-CH_2-O-)_nH$ mit jeweils n = 1 bis 20, vorzugsweise 3 bis 20.

Weiterer Gegenstand der vorliegenden Erfindung ist ebenso die Verwendung erfindungsgemäßer Liganden und/oder Metall-Ligand-Komplexe in Wasch- oder Reinigungsmitteln, insbesondere zur Reinigung textiler Flächengebilde sowie zur Reinigung harter Oberflächen.

Weiterer Gegenstand der vorliegenden Erfindung ist ebenso die Verwendung erfindungsgemäßer Liganden und/oder Metall-Ligand-Komplexe, insbesondere als Hilfsmittel, zur Reinigung textiler Flächengebilde sowie zur Reinigung harter Oberflächen.

Weiterer Gegenstand der vorliegenden Erfindung ist ebenso die Verwendung erfindungsgemäßer Liganden und/oder Metall-Ligand-Komplexe zum Bleichen von Zellstoff und/oder Roh-Baumwolle.

Bei den erfindungsgemäßen Wasch- und Reinigungsmitteln kann es sich um alle denkbaren Reinigungsmittelarten handeln, sowohl um Konzentrate als auch um unverdünnt anzuwendende Mittel, zum Einsatz im kommerziellen Maßstab, in der Waschmaschine oder bei der Hand-Wäsche, beziehungsweise -Reinigung. Dazu gehören beispielsweise Waschmittel für Textilien, Teppiche, oder Naturfasern, für die nach der vorliegenden Erfindung die Bezeichnung Waschmittel verwendet wird. Dazu gehören beispielsweise auch Geschirrspülmittel für Geschirrspülmaschinen oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder; für solche wird nach der vorliegenden Erfindung die Bezeichnung Reinigungsmittel verwendet. Im weiteren Sinne sind auch Sterilisations- und Desinfektionsmittel als Wasch- und Reinigungsmittel im erfindungsgemäßen Sinne anzusehen.

Ausführungsformen der vorliegenden Erfindung umfassen alle nach dem Stand der Technik etablierten und/oder alle zweckmäßigen Darreichungsformen der erfindungsgemäßen Wasch- oder Reinigungsmittel. Dazu zählen beispielsweise feste, pulverförmige, flüssige, gelförmige oder pastöse Mittel, gegebenenfalls auch aus mehreren Phasen, komprimiert oder nicht komprimiert; ferner gehören beispielsweise dazu: Extrudate, Granulate, Tabletten oder Pouches,

sowohl in Großgebinden als auch portionsweise abgepackt.

In einer bevorzugten Ausführungsform enthalten die erfindungsgemäßen Wasch- oder Reinigungsmittel die oben beschriebenen erfindungsgemäßen Bleichkatalysatoren in einer Menge von bis zu 5 Gew.-%, insbesondere von 0,001 Gew.-% bis 1 Gew.-% und besonders bevorzugt von 0,01 Gew.-% bis 0,5 Gew.-%, vor allem von 0,01 bis 0,25 Gew.-%, jeweils bezogen auf das Gesamtgewicht des Wasch- oder Reinigungsmittels.

Neben den erfindungsgemäßen Bleichkatalysatoren können gegebenenfalls zusätzlich auch andere Bleichkatalysatoren in den erfindungsgemäßen Mitteln enthalten sein. Bei diesen Stoffen kann es sich generell um jedes beliebige bleichverstärkende Übergangsmetallsalz beziehungsweise jeden beliebigen Übergangsmetallkomplex handeln. Als Übergangsmetalle kommen hierbei insbesondere Mn, Fe, Co, Ru, Mo, Ti, V oder Cu in unterschiedlichen Oxidationsstufen in Betracht. Als mögliche komplexierende Liganden kommen insbesondere, wie in der Literatur beschrieben, Guanidine, Aminophenole, Aminoxide, Salene, Saldimine, Lactame, monocyclische sowie querverbrückte polycyclische Polyazaalkane, Terpyridine, Dendrimere, Tetraamido-Liganden, Bis- und Tetrakis(pyridylmethyl)alkylamine, sekundäre Amine und Polyoxometallate in Betracht.

In einer bevorzugten Ausführungsform wird als zusätzlicher Bleichkatalysator ein Komplex des Mangans in der Oxidationsstufe II, III, IV oder V eingesetzt, der vorzugsweise einen oder mehrere makrocyclische Liganden mit den Donorfunktionen N, NR, PR, O und/oder S enthält. Vorzugsweise werden hierbei Liganden eingesetzt, die Stickstoff-Donorfunktionen aufweisen. Dabei ist es besonders bevorzugt, zusätzlich einen Bleichkatalysator in den erfindungsgemäßen Mitteln einzusetzen, der als makromolekularen Liganden 1,4,7-Trimethyl-1,4,7-triazacyclononan (Me-TACN), 1,4,7-Triazacyclononan (TACN), 1,5,9-Trimethyl-1,5,9-triazacyclododecan (Me-TACD), 2-Methyl-1,4,7-trimethyl-1,4,7-triazacyclononan (Me/Me-TACN) und/oder 2-Methyl-1,4,7-triazacyclononan (Me/TACN) enthält. Geeignete Mangankomplexe sind beispielsweise $[Mn^{III}_2(\mu\text{-}O)_1(\mu\text{-}OAc)_2(TACN)_2](ClO_4)_2$, $[Mn^{III}Mn^{IV}(\mu\text{-}O)_2(\mu\text{-}OAC)_1(TACN)_2](BPh_4)_2$, $[Mn^{IV}_4(\mu\text{-}O)_6(TACN)_4](ClO_4)_4$, $[Mn^{III}_2(\mu\text{-}O)_1(\mu\text{-}OAc)_2(Me\text{-}TACN)_2](ClO_4)_2$, $[Mn^{III}Mn^{IV}(\mu\text{-}O)_1(\mu\text{-}OAc)_2(Me\text{-}TACN)_2](ClO_4)_3$, $[Mn^{IV}_2(\mu\text{-}O)_3(Me\text{-}TACN)_2](PF_6)_2$ und $[Mn^{IV}_2(\mu\text{-}O)_3(Me/Me\text{-}TACN)_2](PF_6)_2$(OAc = $OC(O)CH_3$).

Auch der zusätzliche Bleichkatalysator ist, soweit eingesetzt, in den erfindungsgemäßen Mitteln vorzugsweise in einer Menge von bis zu 5 Gew.-%, insbesondere von 0,0025 Gew.-% bis 1 Gew.-% und besonders bevorzugt von 0,01 Gew.-% bis 0,25 Gew.-%, jeweils bezogen auf das Gesamtgewicht des Wasch- oder Reinigungsmittels, enthalten.

Des Weiteren sind in den erfindungsgemäßen Wasch- und Reinigungsmitteln vorzugsweise Bleichmittel enthalten, die vorzugsweise das Substrat für die erfindungsgemäßen Bleichkatalysatoren darstellen und/oder liefern. Unter einem Bleichmittel ist in diesem Sinne zum einen Wasserstoffperoxid selbst und zum anderen jede Verbindung, die in wässrigem Medium Wasserstoffperoxid liefert, zu verstehen. Unter den als Bleichmittel dienenden, in Wasser $H_2O_2$ liefernden Verbindungen haben das Natriumpercarbonat, das Natriumperborattetrahydrat und das Natriumperboratmonohydrat besondere Bedeutung. Weitere brauchbare Bleichmittel sind beispielsweise Peroxopyrophosphate, Citratperhydrate sowie $H_2O_2$ liefernde persaure Salze oder Persäuren, wie Persulfate beziehungsweise Perschwefelsäure. Brauchbar ist auch das Harnstoffperoxohydrat Percarbamid, das durch die Formel $H_2N\text{-}CO\text{-}NH_2 \cdot H_2O_2$ beschrieben werden kann. Insbesondere beim Einsatz der Mittel für das Reinigen harter Oberflächen, zum Beispiel beim maschinellen Geschirrspülen, können sie gewünschtenfalls auch Bleichmittel aus der Gruppe der organischen Bleichmittel enthalten, obwohl deren Einsatz prinzipiell auch bei Mitteln für die Textilwäsche möglich ist. Typische organische Bleichmittel sind die Diacylperoxide, wie zum Beispiel Dibenzoylperoxid. Weitere typische organische Bleichmittel sind die Peroxysäuren, wobei als Beispiele besonders die Alkylperoxysäuren und die Arylperoxysäuren genannt werden. Bevorzugte Vertreter sind (a) die Peroxybenzoesäure und ihre ringsubstituierten Derivate, wie Alkylperoxybenzoesäuren, aber auch Peroxy-$\alpha$-Naphthoesäure und Magnesium-monoperphthalat, (b) die aliphatischen oder substituiert aliphatischen Peroxysäuren, wie Peroxylaurinsäure, Peroxystearinsäure, $\varepsilon$-Phthalimidoperoxycapronsäure (Phthalimidoperoxyhexansäure, PAP), o-Carboxybenzamidoperoxycapronsäure, N-Nonenylamidoperadipinsäure und N-Nonenylamidopersuccinate, und (c) aliphatische und araliphatische Peroxydicarbonsäuren, wie 1,12-Diperoxycarbonsäure, 1,9-Diperoxyazelainsäure, Diperoxysebacinsäure, Diperoxybrassylsäure, die Diperoxyphthalsäuren, 2-Decyldiperoxybutan-1,4-disäure, N,N-Terephthaloyl-di(6-aminopercapronsäure).

Als Bleichmittel können auch Chlor oder Brom freisetzende Substanzen eingesetzt werden. Unter den geeigneten Chlor oder Brom freisetzenden Materialien kommen beispielsweise heterozyklische N-Brom- und N-Chloramide, beispielsweise Trichlorisocyanursäure, Tribromisocyanursäure, Dibromisocyanursäure und/oder Dichlorisocyanursäure (DICA) und/oder deren Salze mit Kationen wie Kalium und Natrium in Betracht. Hydantoinverbindungen, wie 1,3-Dichlor-5,5-dimethylhydanthoin sind ebenfalls geeignet.

In einer besonderen erfindungsgemäßen Ausführungsform wird auf den Einsatz von Wasserstoffperoxid liefernden Substanzen verzichtet und als Bleichmittel wird stattdessen Sauerstoff eingesetzt, wobei es sich bei dem Sauerstoff um Luftsauerstoff handeln kann oder um Sauerstoff, der aus einem Sauerstoff liefernden Mittel freigesetzt wird.

Erfindungsgemäß werden Wasch- oder Reinigungsmittel, insbesondere maschinelle Geschirrspülmittel, bevorzugt, die bis zu 45 Gew.-%, insbesondere 1 bis 35 Gew.-%, vorzugsweise 2,5 bis 30 Gew.-%, besonders bevorzugt 3,5 bis 20 Gew.-% und insbesondere 5 bis 15 Gew.-% Bleichmittel, vorzugsweise Natriumpercarbonat, enthalten.

Der Aktivsauerstoffgehalt der Wasch- oder Reinigungsmittel, insbesondere der maschinellen Geschirrspülmittel, beträgt, jeweils bezogen auf das Gesamtgewicht des Mittels, vorzugsweise zwischen 0,4 und 10 Gew.-%, besonders bevorzugt zwischen 0,5 und 8 Gew.-% und insbesondere zwischen 0,6 und 5 Gew.-%. Besonders bevorzugte Mittel weisen einen Aktivsauerstoffgehalt oberhalb 0,3 Gew.-%, bevorzugt oberhalb 0,7 Gew.-%, besonders bevorzugt oberhalb 0,8 Gew.-% und insbesondere oberhalb 1,0 Gew.-% auf.

Alternativ zu als auch gleichzeitig mit den Bleichmitteln können zum Bereitstellen des Wasserstoffperoxids auch Enzyme eingesetzt werden, die ausgehend von anderen Substraten dazu in der Lage sind, Wasserstoffperoxid in situ zu erzeugen. Es handelt sich hierbei um Oxidoreduktasen, die Elektronen von - in der Regel - einem organischen Substrat, etwa der Glucose, auf Sauerstoff als Elektronenakzeptor transferieren können und so die Ausbildung des gewünschten Wasserstoffperoxids in situ ermöglichen. Die Oxidoreduktase kann hierbei zusammen mit dem entsprechenden organischen Substrat eingesetzt werden. Da die zu behandelnden Anschmutzungen jedoch bereits das erforderliche Substrat enthalten können, kann der Einsatz der Oxidoreduktasen gegebenenfalls auch ohne Zusatz des entsprechenden Substrats erfolgen.

Bei der Wasserstoffperoxid erzeugenden Oxidoreduktase handelt es sich vorzugsweise um eine Oxidoreduktase, die Wasserstoffperoxid produziert, indem sie Sauerstoff als Elektronenakzeptor verwendet. Hierbei kommen insbesondere Oxidoreduktasen der EC-Klassen E.C. 1.1.3 (CH-OH als Elektronendonor), E.C. 1.2.3 (Aldehyd oder Oxo-Gruppe als Elektronendonor), E.C. 1.4.3 ($CH-NH_2$ als Donor), E.C. 1.7.3 (N-haltige Gruppe als Donor) und E.C. 1.8.3 (S-haltige Gruppe als Donor) in Betracht, wobei Enzyme der EC-Klasse E.C. 1.1.3 bevorzugt sind.

Bevorzugte Enzyme sind insbesondere ausgewählt aus der Gruppe bestehend aus Malat-Oxidase (EC 1.1.3.3), Glucose-Oxidase (EC 1.1.3.4), Hexose-Oxidase (EC 1.1.3.5), Cholesterin-Oxidase (EC 1.1.3.6), Galactose-Oxidase (EC 1.1.3.9), Pyranose-Oxidase (EC 1.1.3.10), Alkohol-Oxidase (EC 1.1.3.13), Cholin-Oxidase (EC 1.1.3.17, siehe insbesondere WO 04/58955), Oxidasen für langkettige Alkohole (EC 1.1.3.20), Glycerin-3-phosphat-Oxidase (EC 1.1.3.21), Cellobiose-Oxidase (EC 1.1.3.25), Nucleosid-Oxidase (EC 1.1.3.39), D-Mannitol-Oxidase (EC 1.1.3.40), Xylitol-Oxidase (EC 1.1.3.41), Aldehyd-Oxidase (EC 1.2.3.1), Pyruvat-Oxidase (EC 1.2.3.3), Oxalat-Oxidase (EC 1.2.3.4), Glyoxylat-Oxidase (EC 1.2.3.5), Indol-3-acetaldehyd-Oxidase (EC 1.2.3.7), Pyridoxal-Oxidase (EC 1.2.3.8), Arylaldehyd-Oxidase (EC 1.2.3.9), Retinal-Oxidase (EC 1.2.3.11), L-Aminosäure-Oxidase (EC 1.4.3.2), Amin-Oxidase (EC 1.4.3.4, EC 1.4.3.6), L-Glutamat-Oxidase (EC 1.4.3.11), L-Lysin-Oxidase (EC 1.4.3.14), L-Aspartat-Oxidase (EC 1.4.3.16), Tryptophan-alpha,beta-Oxidase (EC 1.4.3.17), Glycin-Oxidase EC 1.4.3.19), Harnstoff-Oxidase (EC 1.7.3.3), Thiol-Oxidase (EC 1.8.3.2), Glutathion-Oxidase (EC 1.8.3.3), Sorbitol-Oxidase sowie aus Enzymen wie etwa in DE102005053529 beschrieben.

Bei der Wasserstoffperoxid produzierenden Oxidoreduktase handelt es sich in einer bevorzugten Ausführungsform um eine, die einen Zucker als Elektronendonor verwendet. Die Wasserstoffperoxid produzierende und Zucker oxidierende Oxidoreduktase ist erfindungsgemäß vorzugsweise ausgewählt aus Glucose-Oxidase (EC 1.1.3.4), Hexose-Oxidase (EC 1.1.3.5), Galactose-Oxidase (EC 1.1.3.9) und Pyranose-Oxidase (EC 1.1.3.10). Besonders bevorzugt ist erfindungsgemäß die Glucose-Oxidase (EC 1.1.3.4).

Vorteilhafterweise werden bei Verwendung einer Wasserstoffperoxid erzeugenden Oxidoreduktase zusätzlich vorzugsweise organische, besonders bevorzugt aromatische, mit den Enzymen wechselwirkende Verbindungen zugegeben, um die Aktivität der betreffenden Oxidoreduktasen zu verstärken (Enhancer) oder um bei stark unterschiedlichen Redoxpotentialen zwischen den oxidierenden Enzymen und den Anschmutzungen den Elektronenfluß zu gewährleisten (Mediatoren).

Die Wasserstoffperoxid produzierende Oxidoreduktase wird in den erfindungsgemäßen Wasch - und Reinigungsmitteln, falls sie verwendet werd, vorzugsweise in einer solchen Menge eingesetzt, dass das gesamte Mittel eine auf die Oxidoreduktase bezogene Enzym-Aktivität von 30 U/g bis 20.000 U/g, insbesondere von 60 U/g bis 15.000 U/g aufweist. Die Einheit 1 U (Unit) entspricht hierbei der Aktivität derjenigen Enzymmenge, die 1 $\mu$mol ihres Substrats bei pH 7 und 25 °C in einer Minute umsetzt.

Das gegebenenfalls bei Verwendung einer solchen Wasserstoffperoxid produzierenden Oxidoreduktase einzusetzende Substrat ergibt sich in der Regel unmittelbar aus der Bezeichnung der jeweiligen Oxidoreduktase.

Erfindungsgemäße Mittel können gegebenenfalls auch Bleichaktivatoren als zusätzlichen Bleichhilfsstoff enthalten.

Neben einem erfindungsgemäßen Bleichkatalysator und den zuvor genannten Bleichmitteln und optional enthaltenen weiteren Bleichhilfsstoffen enthält ein erfindungsgemäßes Wasch- oder Reinigungsmittel gegebenenfalls weitere Inhaltsstoffe wie weitere Enzyme, Enzymstabilisatoren, Tenside, insbesondere nichtionische, anionische, kationische und/oder amphotere Tenside, Gerüststoffe (Builder, organische Cobuilder), wasch- und reinigungsaktive Polymere, Lösungsmittel, Verdicker, Sequestrierungsmittel, Elektrolyte, Acidifizierungsmittel, optische Aufheller, Vergrauungsinhibitoren, Glaskorrosionsinhibitoren, Korrosionsinhibitoren, Farbübertragungsinhibitoren, Schauminhibitoren, Desintegrationshilfsstoffe, Abrasivstoffe, Farbstoffe, Duftstoffe, mikrobielle Wirkstoffe, UV-Absorbenzien, synthetische Knitterschutzmittel, Antistatika, sogenannte Soil-Release-Wirkstoffe bzw. Soil-Repellents und/oder Treibmittel.

Als besonders vorteilhafte Bleichkatalysatorgranulate haben sich solche herausgestellt, die bezogen auf das Gesamtgewicht des Granulats

a) 0,1 bis 30 Gew.-% eines erfindungsgemäßen Bleichkatalysators sowie gegebenenfalls weiteren Bleichkatalysator,
b) 10 bis 99 Gew.-% eines Trägermaterials, sowie
c) 0,9 bis 5 Gew.-% eines Bindemittels aus der Gruppe der organischen Polymere enthalten.

Der gegebenenfalls einzusetzende weitere Bleichkatalysator gemäß a) ist hierbei vorzugsweise ausgewählt aus den bereits zuvor genannten weiteren Bleichkatalysatoren.

Als Trägermaterial b) eignen sich grundsätzlich alle in Wasch- und Reinigungsmitteln einsetzbaren mit den übrigen Inhaltsstoffen kompatiblen Substanzen oder Substanzgemische, insbesondere die bereits zuvor aufgezählten Gerüststoffe, vor allem die Carbonate, einschließlich der Hydrogencarbonate, die Sulfate, die Chloride, die Silikate und die Phosphate. Als Trägermaterial eignen sich hierbei insbesondere Alkalimetallcarbonate, Alkalimetallhydrogencarbonate, Alkalimetallsesquicarbonate, Alkalisilikate, Alkalimetasilikate, Alkallphosphate und Mischungen dieser Stoffe, wobei im Sinne dieser Erfindung bevorzugt die Alkalicarbonate, insbesondere Natriumcarbonat, Natriumhydrogencarbonat oder Natriumsesquicarbonat, und/oder Alkaliphosphate eingesetzt werden. In einer besonders bevorzugten Ausführungsform wird als Trägermaterial das Pentanatriumtriphosphat, $Na_5P_3O_{10}$ (Natriumtripolyphosphat) oder das entsprechende Kaliumsalz Pentakaliumtriphosphat, $K_5P_3O_{10}$(Kaliumtripolyphosphat) eingesetzt.

Der Gewichtsanteil des Trägermaterials b) am Gesamtgewicht der Bleichkatalysatorgranulate kann in eingangs angegebenen Grenzen variiert werden, wobei sich hinsichtlich der Verarbeitbarkeit und der tatsächlichen Bleichleistung nach der Konfektionierung mit weiteren wasch- und reinigungsaktiven Inhaltsstoffen insbesondere Gewichtsanteile oberhalb 20 Gew.-%, vorzugsweise oberhalb 40 Gew.-% und insbesondere oberhalb 60 Gew.-% als vorteilhaft erwiesen haben. Folglich werden im Rahmen der vorliegenden Anmeldung Bleichkatalysatorgranulate bevorzugt, bei denen der Gewichtsanteil des Trägermaterials b) am Gesamtgewicht des Granulats 20 bis 99 Gew.-%, vorzugsweise zwischen 40 und 95 Gew.-% und insbesondere zwischen 60 und 90 Gew.-% beträgt.

Als dritten Inhaltsstoff enthalten die erfindungsgemäßen Bleichaktivatorgranulate ein Bindemittel c) aus der Gruppe der organischen Polymere. Die Polymere können nichtionischer, anionscher, kationischer oder amphoterer Natur sein. Natürliche Polymere und modifizierte Polymere natürlichen Ursprungs sind ebenso einsetzbar wie synthetische Polymere.

Zur Gruppe der mit besonderem Vorzug als Bindemittel c) eingesetzten nichtionischen Polymere zählen Polyvinylalkohole, acetalisierte Polyvinylalkohole, Polyvinylpyrrolidone und Polyalkylenglykole, insbesondere Polyethylenoxide. Bevorzugte Polyvinylalkohole und acetalisierte Polyvinylalkohole weisen Molekulargewicht im Bereich von 10.000 bis 100.000 $gmol^{-1}$, vorzugsweise von 11.000 bis 90.000 $gmol^{-1}$, besonders bevorzugt von 12.000 bis 80.000 $gmol^{-1}$ und insbesondere von 13.000 bis 70.000 $gmol^{-1}$ auf. Bevorzugte Polyethylenoxide haben Molmassen im Bereich von ca. 200 bis 5.000.000 g/mol, entsprechend Polymerisationsgraden n von ca. 5 bis >100.000.

Zu der Gruppe der mit besonderem Vorzug als Bindemittel c) eingesetzten anionischen Polymere gehören insbesondere homo- oder copolymere Polycarboxylate, Polyacrylsäuren und Polymethacrylsäuren, insbesondere solche, die bereits zuvor als für Wasch- und Reinigungsmittel brauchbare organische Gerüstsubstanzen genannt wurden, sowie Sulfonsäuregruppen-haltige Polymere, insbesondere solche, die bereits zuvor als brauchbare Enthärter genannt wurden.

Hinsichtlich der Gruppe der mit besonderem Vorzug als Bindemittel c) eingesetzten kationischen und amphoteren Polymere wird auf die bereits zuvor als wasch- und reinigungsaktive Polymere aufgezählten Polymere verwiesen.

In erfindungsgemäß bevorzugten Bleichkatalysatorgranulaten beträgt der Gewichtsanteil des Bindemittels c) am Gesamtgewicht des Granulats zwischen 0,2 und 4,5 Gew.-%, bevorzugt zwischen 0,5 und 4,0 Gew.-% und insbesondere zwischen 1,0 und 4,0 Gew.-%.

Die Bleichkatalysatorgranulate besitzen vorzugsweise eine mittlere Teilchengröße zwischen 0,1 und 1,0 mm, besonders bevorzugt zwischen 0,2 und 0,8 mm und insbesondere zwischen 0,3 und 0,7 mm, wobei der Gewichtsanteil der Teilchen mit einer Teilchengröße unterhalb 0,1 mm vorzugsweise mindestens 4 Gew.-%, besonders bevorzugt mindestens 6 Gew.-% und insbesondere mindestens 8 Gew.-%, jedoch gleichzeitig vorzugsweise maximal 80 Gew.-%, besonders bevorzugt maximal 60 Gew.-% und insbesondere maximal 40 Gew.-% beträgt, und der Gewichtsanteil der Teilchen mit einer Teilchengröße zwischen 0,2 und 0,8 mm vorzugsweise zwischen 30 und 70 Gew.-%, besonders bevorzugt zwischen 45 und 65 Gew.-% und insbesondere zwischen 40 und 60 Gew.-% beträgt

Außer dem Bleichkatalysator können auch Enzyme oder andere Inhaltsstoffe, insbesondere sensitive, auf die zuvor beschriebene Art und Weise konfektioniert werden.

Einen eigenen Erfindungsgegenstand stellen Verfahren zur Reinigung von Textilien oder von harten Oberflächen dar, bei denen wenigstens in einem der Verfahrensschritte ein erfindungsgemäßer Bleichkatalysator verwendet wird.

Hierunter fallen sowohl manuelle als auch maschinelle Verfahren. Ausführungsformen stellen beispielsweise die Handwäsche, die manuelle Entfernung von Flecken von Textilien oder von harten Oberflächen oder die Verwendung im Zusammenhang mit einem maschinellen Verfahren dar, wobei maschinelle Verfahren, insbesondere zur Reinigung von Textilien, aufgrund ihrer präziseren Steuerbarkeit, was beispielsweise die eingesetzten Mengen und Einwirkzeiten

angeht, bevorzugt sind. Entsprechend bevorzugt gelten für diese Verwendungen die oben angeführten Konzentrationsbereiche.

Die Reinigung der textilen Flächengebilde erfolgt vorzugsweise bei Temperaturen von 20 - 95°C, in einer bevorzugten Ausführungsform bei Temperaturen von 20 - 60 °C, insbesondere bei Temperaturen von 20 - 40°C, sowie vorzugsweise bei einem pH-Wert von 5-12, insbesondere von 8-11.

Verfahren zur Reinigung von Textilien zeichnen sich im allgemeinen dadurch aus, dass in mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Waschmittel oder einer Lösung dieses Mittels behandelt wird. Das gleiche gilt für Verfahren zur Reinigung von allen anderen Materialien als Textilien, welche unter dem Begriff "harte Oberflächen" zusammengefasst werden. Alle denkbaren Wasch- oder Reinigungsverfahren können in wenigstens einem der Verfahrensschritte um einen erfindungsgemäßen Bleichkatalysator bereichert werden, und stellen dann Ausführungsformen der vorliegenden Erfindung dar.

In einer bevorzugten Ausführungsform dieser Verwendung werden die erfindungsgemäßen Bleichkatalysatoren hierbei im Rahmen einer der oben ausgeführten Rezepturen für erfindungsgemäße Mittel, vorzugsweise Wasch- beziehungsweise Reinigungsmittel, bereitgestellt.

Ein weiterer Gegenstand der vorliegenden Erfindung ist auch ein Erzeugnis, enthaltend eine erfindungsgemäße Zusammensetzung bzw. ein erfindungsgemäßes Wasch- oder Reinigungsmittel, insbesondere einen erfindungsgemäßen Reiniger für harte Oberflächen, und einen Sprühspender. Bei dem Erzeugnis kann es sich hierbei sowohl um ein Einkammer- als auch um ein Mehrkammerbehältnis, insbesondere ein Zweikammerbehältnis handeln. Bevorzugt ist der Sprühspender hierbei ein manuell aktivierter Sprühspender, insbesondere ausgewählt aus der Gruppe umfassend Aerosolsprühspender (Druckgasbehälter; auch u.a. als Spraydose bezeichnet), selbst Druck aufbauende Sprühspender, Pumpsprühspender und Triggersprühspender, insbesondere Pumpsprühspender und Triggersprühspender mit einem Behälter aus transparentem Polyethylen oder Polyethylenterephthalat. Sprühspender werden ausführlicher in der WO 96/04940 (Procter & Gamble) und den darin zu Sprühspendern zitierten US-Patenten, auf die in dieser Hinsicht sämtlich Bezug genommen und deren Inhalt hiermit in diese Anmeldung aufgenommen wird, beschrieben. Triggersprühspender und Pumpzerstäuber besitzen gegenüber Druckgasbehältern den Vorteil, daß kein Treibmittel eingesetzt werden muß. Durch geeignete, partikelgängige Aufsätze, Düsen etc. (sog. "nozzle-Ventile") auf dem Sprühspender kann gegebenenfalls enthaltenes Enzym in dieser Ausführungsform optional auch in auf Partikeln immobilisierter Form dem Mittel beigefügt werden und so als Reinigungsschaum dosiert werden.

Erfindungsgemäß besonders bevorzugte maschinelle Geschirrspülmittel umfassen

- 5 bis 70 Gew.-%, vorzugsweise 10 bis 60 Gew.-% und insbesondere 20 bis 50 Gew.-% Gerüstoff(e), mit Ausnahme wasch- und reinigungsaktiver Polymere;
- 2 bis 28 Gew.-%, vorzugsweise 4 bis 20 Gew.-% und insbesondere 6 bis 15 Gew.-% wasch - und reinigungsaktive Polymere;
- 0,5 bis 10 Gew.-%, vorzugsweise 1 bis 8 Gew.-% und insbesondere 2 bis 6 Gew.-% Tensid(e), vorzugsweise nichtionische(s) und/oder amphotere(s) Tensid(e);
- 0,5 bis 8 Gew.-%, vorzugsweise 1 bis 7 Gew.-% und insbesondere 2 bis 6 Gew.-% Enzym(e);
- 2 bis 20 Gew.-%, vorzugsweise 4 bis 15 Gew.-% und insbesondere 6 bis 12 Gew.-% Bleichmittel;
- 0,01 bis 5 Gew.-%, vorzugsweise 0,02 bis 4 Gew.-% und insbesondere 0,05 bis 3 Gew.-% erfindungsgemäße Bleichkatalysatoren; sowie gegebenenfalls
- 0,01 bis 5 Gew.-%, vorzugsweise 0,02 bis 4 Gew.-% und insbesondere 0,05 bis 3 Gew.-% weitere Bleichkatalysatoren.

Ganz besonders bevorzugte maschinelle Geschirrspülmittel umfassen

- 5 bis 70 Gew.-%, vorzugsweise 10 bis 60 Gew.-% und insbesondere 20 bis 50 Gew.-% Phosphate;
- 2 bis 28 Gew.-%, vorzugsweise 4 bis 20 Gew.-% und insbesondere 6 bis 15 Gew.-% wasch - und reinigungsaktive Polymere;
- 0,5 bis 10 Gew.-%, vorzugsweise 1 bis 8 Gew.-% und insbesondere 2 bis 6 Gew.-% nichtionische(s) Tensid(e);
- 0,5 bis 8 Gew.-%, vorzugsweise 1, bis 7 Gew.-% und insbesondere 2 bis 6 Gew.-% Enzym(e) ausgewählt aus Amylasen, Proteasen und Amadoriasen;
- 2 bis 20 Gew.-%, vorzugsweise 4 bis 15 Gew.-% und insbesondere 6 bis 12 Gew.-% Percarbonat;
- 0,01 bis 5 Gew.-%, vorzugsweise 0,02 bis 4 Gew.-% und insbesondere 0,05 bis 3 Gew.-% erfindungsgemäße Bleichkatalysatoren; sowie gegebenenfalls
- 0,01 bis 5 Gew.-%, vorzugsweise 0,02 bis 4 Gew.-% und insbesondere 0,05 bis 3 Gew.-% weitere Bleichkatalysatoren.

Die Konfektionierung erfindungsgemäßer maschineller Geschirrspülmittel kann in unterschiedlicher Weise erfolgen. Die erfindungsgemäßen Mittel können in fester oder flüssiger sowie als Kombination fester und flüssiger Angebotsformen vorliegen.

Als feste Angebotsformen eignen sich insbesondere Pulver, Granulate, Extrudate oder Kompaktate, insbesondere Tabletten. Die flüssigen Angebotsformen auf Basis von Wasser und/oder organischen Lösungsmitteln können verdickt, in Form von Gelen vorliegen.

Erfindungsgemäße Mittel können in Form einphasiger oder mehrphasiger Produkte konfektioniert werden. Bevorzugt werden insbesondere maschinelle Geschirrspülmittel mit einer, zwei, drei oder vier Phasen. Maschinelle Geschirrspülmittel, dadurch gekennzeichnet, dass es in Form einer vorgefertigten Dosiereinheit mit zwei oder mehr Phasen vorliegt, werden besonders bevorzugt.

Die einzelnen Phasen mehrphasiger Mittel können die gleiche oder unterschiedliche Aggregatzustände aufweisen. Bevorzugt werden insbesondere maschinelle Geschirrspülmittel, die mindestens zwei unterschiedliche feste Phasen und/oder mindestens zwei flüssige Phasen und/oder mindestens eine feste und mindestens eine feste Phase aufweisen.

Erfindungsgemäße maschinelle Geschirrspülmittel werden vorzugsweise zu Dosiereinheiten vorkonfektioniert. Diese Dosiereinheiten umfassen vorzugsweise die für einen Reinigungsgang notwendige Menge an wasch- oder reinigungsaktiven Substanzen. Bevorzugte Dosiereinheiten weisen ein Gewicht zwischen 12 und 30 g, bevorzugt zwischen 14 und 26 g und insbesondere zwischen 16 und 22 g auf.

Das Volumen der vorgenannten Dosiereinheiten sowie deren Raumform sind mit besonderem Vorzug so gewählt, dass eine Dosierbarkeit der vorkonfektionierten Einheiten über die Dosierkammer einer Geschirrspülmaschine gewährleistet ist. Das Volumen der Dosiereinheit beträgt daher bevorzugt zwischen 10 und 35 ml, vorzugsweise zwischen 12 und 30 ml und insbesondere zwischen 15 und 25 ml.

Die erfindungsgemäßen maschinellen Geschirrspülmittel, insbesondere die vorgefertigten Dosiereinheiten weisen mit besonderem Vorzug eine wasserlösliche Umhüllung auf.

Die nachfolgenden Beispiele erläutern die Erfindung weiter, ohne sie darauf zu beschränken.

Ausführungsbeispiele

Beispiel 1: Darstellung von 1,10-Bis(2-hydroxyphenyl)phenanthrolin *H2(bpphen):*

2,9-Bis(2-methoxyphenyl)-1,10-phenantrolin[1]

$$2\ C_7H_7BrO + 2\ Li \rightarrow 2\ C_7H_7OLi$$

$$2\ C_7H_7OLi + C_{12}H_6N_2 \rightarrow C_{26}H_{20}N_2O_2$$

Es werden 60 g (0.32 mol) Bromanisol in 50 mL Ether gelöst und bei 0°C langsam zu einer Suspension von 8.9 g (1.28 mol) Lithium in 50 mL Ether getropft. Das Reaktionsgemisch wird anschließend innerhalb einer Stunde auf RT erwärmt und dann für eine Stunde am Rückfluss gehalten. Nach Abkühlen wird überschüssiges Lithium über eine Umkehrfritte (Celite) abgetrennt und das Filtrat im Vakuum von LM befreit. Der verbleibende Rückstand wird in 100 mL Toluol aufgenommen und zu der entstehenden Suspension eines nahezu farblosen Feststoffes in gelblicher Lösung bei 0°C eine Lösung von 4.56 g (23 mmol) 1,10-Phenantrolin * $H_2O$ in 150 mL Toluol getropft. Die entstehende blutrote Lösung wird
auf RT erwärmt und weitere 5 h am Rückfluss gehalten. Nach Abkühlen wird mit 50 mL Wasser gequencht und drei Mal mit je 50 mL Dichlormethan extrahiert. Es werden 120 g $MnO_2$ zugegeben, die entstehende Suspension für 4 h bei RT gerührt, durch Celite filtriert und das Filtrat von LM befreit. Nach Säulenchromatographie (Kiselgel, Eluent: Ether/DCM, 20: 1) wird das Produkt als fahlgelber Feststoff erhalten.
$^1$H-NMR (300 MHz, $CDCl_3$): $\delta$ = 3.93 (s, 6H), 7.07 (d, 2H, $^3J$ = 8.31 Hz), 7.19 (t, 2H, $^3J$ = 7.56 Hz), 7.47 (t, 2H, $^3J$ = 7.75 Hz), 7.90 (s, 2H), 8.29 (dd, 2H, $^3J$ = 7.55 Hz, $^5j$ = 1.32 Hz), 8.44 (m, 4H) ppm.
Ausbeute: 2.36 g (26% d.Th.)
2,9-Bis(2-hydroxyphenyl)-1,10-phenantrolin
$C_{26}H_{20}N_2O_2 \rightarrow C_{24}H_{16}N_2O_2$

Es werden 2.6 g (6 mmol) 2,9-Bis(2-methoxyphenyl)-1,10-phenantrolin in 80 mL DMF gelöst und 2.0 g NaSEt (4 eq.) zugegeben. Das Reaktionsgemisch wird für 4 h am Rückfluss gehalten und nach Abkühlen mit einem Gemisch von 100 mL 20%iger $H_2O_2$- und 100 mL 10%iger NaOH-Lösung versetzt, wobei sich sofort ein gelber, voluminöser Niederschlag bildet.

Nach 20 min. rühren bei RT wird vorsichtig mit verd. $H_2SO_4$(aq) leicht angesäuert (pH = ~6) und fünf Mal mit je 100 mL DCM extrahiert. Die vereinigten org. Phasen werden mit Brine gewaschen, über NaSO4 getrocknet und auf ca. 100 mL eingeengt. Durch Zugabe von 400 mL EtOH und abkühlen auf -30°C lässt sich das Produkt als gelbes Pulver ausfällen und durch Filtration abtrennen.
Ausbeute: 1.27 g (58% d.Th.)
$^1$H-NMR (300 MHz, $DMSO_3$): $\delta$ = 7.06 (m, 4H), 7.42 (t, 2H, $^3J$ = 7.27 Hz), 8.07 (s, 2H, 8.27 (d, 2H, $^3J$ = 7.93 Hz), 8.60 (d, 2H, $^3J$ = 8.68 Hz), 8.69 (d, 2H, $^3J$ = 8.68 Hz), 13.92 (s, 2H) ppm.
-> Synthese des Komplexes [Mn(bpphen)]Cl siehe [1]
[1] S. Routier, V. Joanny, A. Zaparucha, H. Vezin, J.-P. Catteau, J.-L. Bernier, C. Bailly, J. Chem. Soc., Perkin Trans. 2, 1998, 863-868.

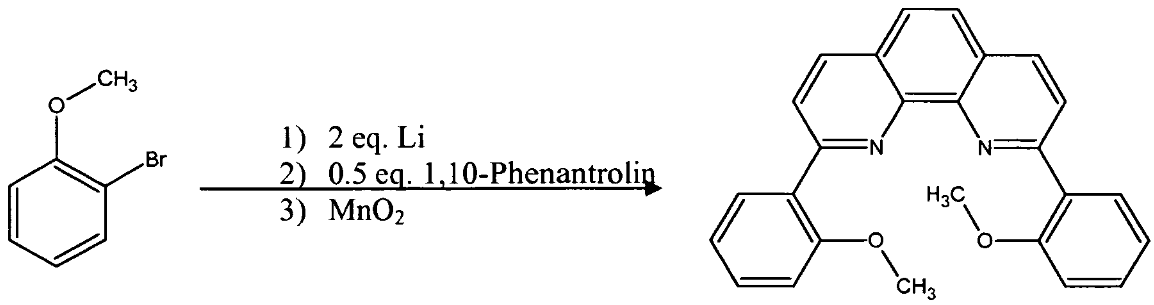


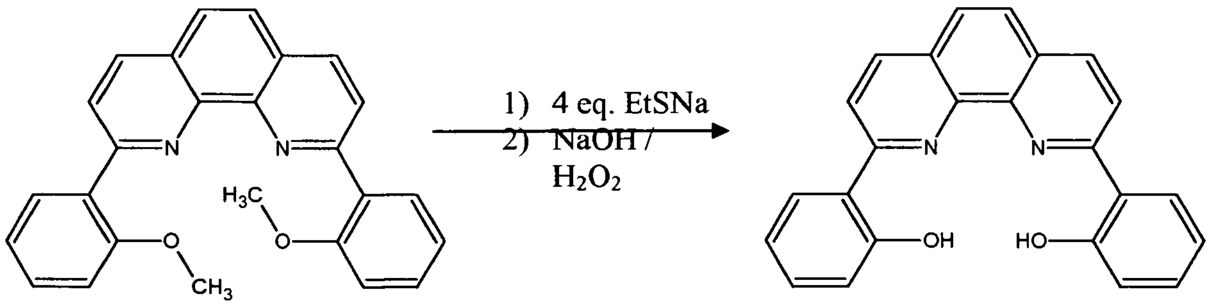


Beispiel 2: Waschtests in Modellwaschanlage

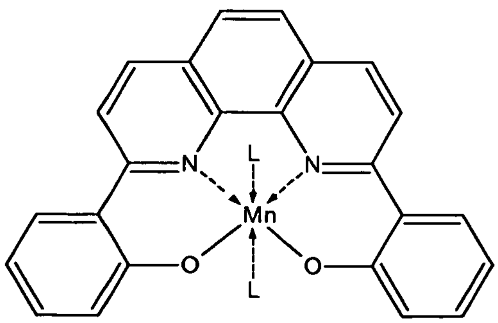

Mn-bpphen

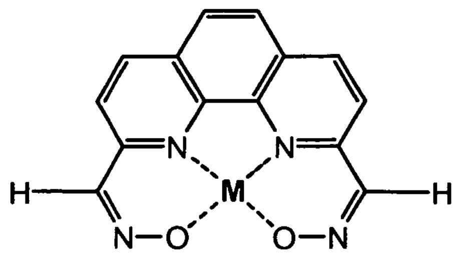

M-bfophen mit M = Mn

Der Waschtest wird in einer temperierbaren Multirührapparatur durchgeführt.

Als Testgefäße dienen 1l Bechergläser, in denen eine Vorrichtung zum mechanischen Rühren der Waschflotte existiert. Die Rührmechanik ist so ausgelegt, dass zum einen alle Bechergläser mit der selben Geschwindigkeit gerührt werden und zum anderen die Rührrichtung periodisch wechselt. Die Beladung der Waschkammern erfolgt mit ca. 16 g Ballastwäsche und ca. 6 g angeschmutztem Gewebe (die Gewebestücke werden in quadratische Form von ca. 6 cm Kantenlänge geschnitten und bestehen aus Baumwolle). Alle Testgewebe werden von der Firma CFT B.V. (Niederlande)

hergestellt.

Bei dem angeschmutzten Gewebe handelt es sich um folgende bleichrelevante Testsubstrate:

| | |
|---|---|
| CS-103 | Rotwein |
| CS-3 | Rotwein gealtert |
| BC-1 | Tee |
| BC-3 | Tee |
| CS-15 | Heidelbeersaft |

Aus diesen 5 Testgeweben wird ein Satz von acht angeschmutzten Gewebestücken für die Tests zusammengestellt. Dies bedeutet, dass drei Anschmutzungen doppelt im Test vertreten sind.

Um die Bleichleistung zu ermitteln wird der Tristimulusvalue Y (Helligkeitswert) der gebleichten Gewebe ermittelt und mit den Referenzproben verglichen. Der Tristimulusvalue Y wird aus dem gemessenen L-Wert über folgende mathematische Beziehung berechnet:

$$L = 116(Y/Y_n)^{1/3} - 16$$

Die Messung der L-Werte wird mit einem Minolta Spektrophotometer CM-508d durchgeführt. Es werden grundsätzlich zwei Testszenarien für Waschtests zur Ermittlung der Bleichaktivität angewendet. Zum einen Waschtests mit einer kompletten Waschmittelrezeptur ohne TAED (Waschtest mit Vollwaschmittel ohne TAED) und zum anderen ein vereinfachter Waschtest, der nur Wasserstoffperoxid und Tenside enthält ($H_2O_2$-Test). Folgende Testparameter kommen beim Waschtest mit Vollwaschmittel ohne TAED zur Anwendung:

Volumen der Waschmittellösung: 750 ml
Menge Waschmittel mit TAED: (100 g VWM pro 16 l Flotte, folglich 4,69 g pro 750 ml)
Menge Waschmittel ohne TAED: 4,55 g pro 750 ml
Metallkatalysator: 0,0086 mmol pro Übergangsmetallatom

Temperatur: 30 °C
Waschzeit: 60 min
Spülvolumen: 500 ml
Spülzeit: 15 min
Wasserqualität: künstl. gehärtetes VE-Wasser mit $CaCl_2$ x 2 $H_2O$ (8,73 g pro 25 l) und $MgCl_2$ x 6 $H_2O$ (2,42 g pro 25 l) = 16° dH)
pH-Wert 10,5 (Carbonatpufferlösung)

Die Waschergebnisse für die verschiedenen Übergangsmetallkomplexe in Vollwaschmittel ohne TAED sind in folgender Tabelle gezeigt. Als Vergleichswerte sind der Wert für Vollwaschmittel ohne TAED und ohne Übergangsmetallkomplexe ("ohne Katalysator") sowie der Wert für Vollwaschmittel mit TAED angegeben.

Tabelle 1: Waschtests mit Vollwaschmittel ohne TAED

| | |
|---|---|
| Mn-bpphen | 75 |
| Mn-bfophen | 74 |
| ohne Katalysator | 71,7 |
| TAED | 75,8 |

Folgende Testparameter kommen beim vereinfachten Waschtest ($H_2O_2$-Test) zur Anwendung:

Volumen der Waschmittellösung: 750 ml
Menge $H_2O_2$: 10 mmol pro l
Tenside: LAS = 0,58 g; LT07 = 0,12 g
Metallkatalysator: 0,0086 mmol pro Übergangsmetallatom

Temperatur: 30 °C
Waschzeit: 60 min
Wasserqualität: VE-Wasser
pH-Wert 10,5 (Carbonatpufferlösung)

Die Waschergebnisse für die verschiedenen Übergangsmetallkomplexe nach dem vereinfachten Waschtest ($H_2O_2$-Test) sind in folgender Tabelle gezeigt.

Tabelle 2: Vereinfachter Waschtest ($H_2O_2$-Test)

| | |
|---|---|
| Mn-bpphen | 75 |
| M-bfophen | 75 |

## Patentansprüche

1. Wasch- oder Reinigungsmittel enthaltend Liganden oder Metall-Ligand-Komplexe von Liganden der allgemeinen Formel (I)

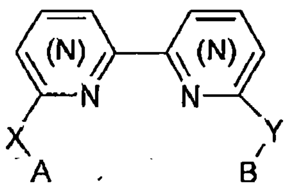
(I),

wobei
X und Y unabhängig voneinander für Ethyl, Ethenyl, -CH=N-, -C($C_{1-18}$-Alkyl)=N-, Cycloalkyl, Cycloheteroalkyl, $C_{6-10}$-Aryl oder Heteroaryl stehen,
A und B unabhängig voneinander für OH, SH, $NH_2$, NH($C_{1-18}$-Alkyl) oder N($C_{1-18}$-Alkyl)$_2$ stehen,
wobei A durch X und B durch Y über 2 Atome mit dem Biheteroaryl-Rest verknüpft sind, wobei (N) bedeutet, dass optional ein oder zwei CH-Gruppen des entsprechenden Arytrests durch N ersetzt sein können,
und wobei das so gebildete Gerüst auch verbrückt sowie ein- oder mehrfach substituiert sein kann.

2. Wasch- oder Reinigungsmittel nach Anspruch 1, **dadurch gekennzeichnet, dass** es sich bei den Liganden um Liganden der allgemeinen Formel (II)

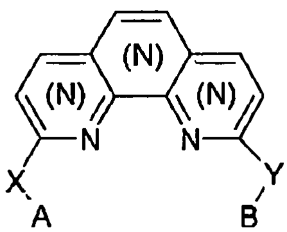
(II)

handelt,
wobei das so gebildete Gerüst auch ein- oder mehrfach substituiert sein kann.

3. Wasch- oder Reinigungsmittel nach Anspruch 1 oder 2, **dadurch gekennzeichnet, dass** X und Y unabhängig voneinander für $C_{6-10}$-Aryl oder Heteroaryl stehen und A und B für OH oder SH stehen.

4. Wasch- oder Reinigungsmittel nach einem der Ansprüche 1 bis 3, **dadurch gekennzeichnet, dass** X und Y ausgewählt sind aus gegebenenfalls substituiertem Benzol, Naphthalin, Pyrrol, Furan, Thiophen, Pyridin, Pyrimidin,

Pyrazin, Pyron, Pyridon, Purin, Triazin, Imidazol, Thiazol, Oxazol, Indol, Chinolin, Isochinolin, Benzimidazol, Benzthiazol oder Benzoxazol.

5. Wasch- oder Reinigungsmittel nach einem der Ansprüche 1, 3 oder 4, **dadurch gekennzeichnet, dass** es sich bei den Liganden um Liganden der allgemeinen Formel (III)

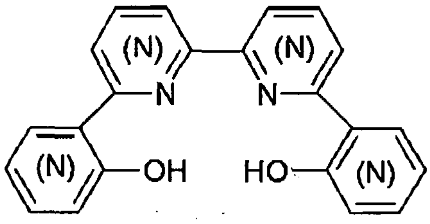

(III)

handelt,
wobei das so gebildete Gerüst auch verbrückt sowie ein- oder mehrfach substituiert sein kann.

6. Wasch- oder Reinigungsmittel nach Anspruch 5, **dadurch gekennzeichnet, dass** es sich bei den Liganden um Liganden der allgemeinen Formel (IV)

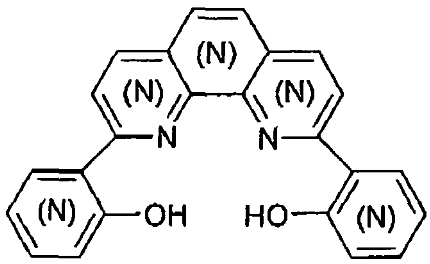

(IV)

handelt,
wobei das Gerüst auch ein- oder mehrfach substituiert sein kann.

7. Wasch- oder Reinigungsmittel nach Anspruch 6, **dadurch gekennzeichnet, dass** es sich bei den Liganden um Liganden der allgemeinen Formel (V)

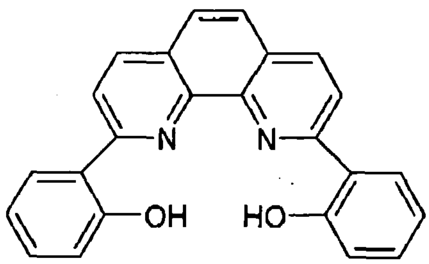

(V)

handelt,
wobei das Gerüst auch ein- oder mehrfach substituiert sein kann.

8. Wasch- oder Reinigungsmittel nach einem der Ansprüche 1 bis 7, **dadurch gekennzeichnet, dass** das Gerüst des Liganden mindestens einen Rest umfassend eine Gruppe ausgewählt aus Ammonium, Hydroxycarbonyl, Alkoxycarbonyl, Aryloxyrarbonyl, Amidocarbonyl, Halogen, Nitro, Sulfato, Sulfo, Amidosulfo, Phosphato, Phosphono, Amidophosphono, Hydroxy, Alkoxy, Amino und Polyoxyethylen als Substituenten trägt.

9. Wasch- oder Reinigungsmittel nach einem der Ansprüche 1 bis 8, **dadurch gekennzeichnet, dass** das Metall des

Metall-Ligand-Komplexes ausgewählt ist aus Ag, Al, Ce, Co, Cu, Fe, Mo, Mn; Ni, Pb, Re, Ti, V und Zn in beliebigen Oxidationsstufen.

**10.** Verwendung von Wasch- und Reinigungsmitteln nach einem der Ansprüche 1 bis 9 zur Reinigung textiler Flächengebilde.

**11.** Verwendung von Wasch- und Reinigungsmitteln nach einem der Ansprüche 1 bis 9 zur Reinigung harter Oberflächen.

**12.** Verwendung von Liganden und/oder Ligand-Metall-Komplexen von Liganden der allgemeinen Formel (I)

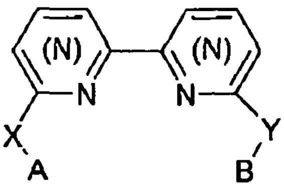
(I),

wobei
X und Y unabhängig voneinander für Ethyl, Ethenyl, -CH=N-, -C($C_{1-18}$-Alkyl)=N-, Cycloalkyl, Cycloheteroalkyl, $C_{6-10}$-Aryl oder Heteroaryl stehen,
A und B unabhängig voneinander für OH, SH, $NH_2$, NH($C_{1-18}$-Alkyl) oder N($C_{1-18}$-Alkyl)$_2$ stehen,
wobei A durch X und B durch Y über 2 Atome mit dem Biheleroaryl-Rest verknüpft sind, wobei (N) bedeutet, dass optional ein oder zwei CH-Gruppen des entsprechenden Arylrests durch N ersetzt sein können,
und wobei das so gebildete Gerüst auch verbrückt sowie ein- oder mehrfach substituiert sein kann,
zur Reinigung textiler Flächengebilde.

**13.** Verwendung von Liganden und/oder Ligand-Metall-Komplexen von Liganden der allgemeinen Formel (I)

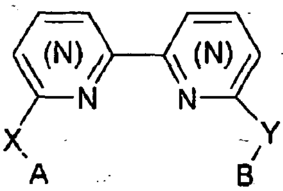
(I),

wobei
X und Y unabhängig voneinander für Ethyl, Ethenyl, -CH=N-, -C($C_{1-16}$-Alkyl)=N-, Cycloalkyl, Cycloheteroalkyl, $C_{6-10}$-Aryl oder Heteroaryl stehen,
A und B unabhängig voneinander für OH, SH, $NH_2$, NH($C_{1-18}$-Alkyl) oder N($C_{1-18}$-Alkyl)$_2$ stehen,
wobei A durch X und B durch Y über 2 Atome mit dem Biheleroaryl-Rest verknüpft sind, wobei (N) bedeutet, dass optional ein oder zwei CH-Gruppen des entsprechenden Arylrests durch N ersetzt sein können,
und wobei das so gebildete Gerüst auch verbrückt sowie ein- oder mehrfach substituiert sein kann,
zur Reinigung harter Oberflächen.

**14.** Verwendung von Liganden und/oder Ligand-Metall-Komplexen von Liganden der allgemeinen Formel (I)

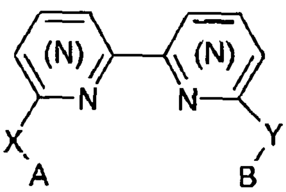
(I),

wobei

X und Y unabhängig voneinander für Ethyl, Ethenyl, -CH=N-, -C($C_{1-18}$-Alkyl)=N-, Cycloalkyl, Cycloheteroalkyl, $C_{6-10}$-Aryl oder Heteroaryl stehen,
A und B unabhängig voneinander für OH, SH, $NH_2$, NH($C_{1-18}$-Alkyl) oder N($C_{1-18}$-Alkyl)$_2$ stehen,
wobei A durch X und B durch Y über 2 Atome mit dem Biheteroaryl-Rest verknüpft sind, wobei (N) bedeutet, dass optional ein oder zwei CH-Gruppen des entsprechenden Arylrests durch N ersetzt sein können,
und wobei das so gebildete Gerüst auch verbrückt sowie ein- oder mehrfach substituiert sein kann,
zum Bleichen von Zellstoff und/oder Roh-Baumwolle.

## Claims

1. A washing or cleaning agent containing ligands or metal-ligand complexes of ligands of the general formula (I)

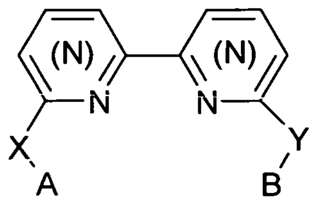

(I),

in which
X and Y mutually independently denote ethyl, ethenyl, -CH=N-, -C($C_{1-18}$-alkyl)=N-, cycloalkyl, cycloheteroalkyl, $C_{6-10}$ aryl or heteroaryl, A and B mutually independently denote OH, SH, $NH_2$, NH($C_{1-18}$-alkyl) or -N($C_{1-18}$-alkyl)$_2$,
in which A is linked by X and B by Y to the biheteroaryl residue via 2 atoms,
in which (N) means that optionally one or two CH groups of the corresponding aryl residue may be replaced by N,
and in which a so formed skeleton can also be bridged as well as mono- or polysubstituted.

2. The washing or cleaning agent according to claim 1, **characterised in that** the ligands comprise ligands of the general formula (II)

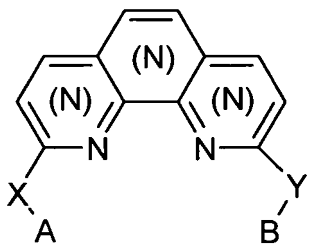

(II)

in which the so formed skeleton can also be mono- or polysubstituted.

3. The washing or cleaning agent according to claim 1 or claim 2,
**characterised in that**
X and Y mutually independently denote $C_{6-10}$ aryl or heteroaryl and
A and B denote OH or SH.

4. The washing or cleaning agent according to any one of claims 1 to 3, **characterised in that** X and Y are selected from optionally substituted benzene, naphthalene, pyrrole, furan, thiophene, pyridine, pyrimidine, pyrazine, pyrone, pyridone, purine, triazine, imidazole, thiazole, oxazole, indole, quinoline, isoquinoline, benzimidazole, benzothiazole or benzoxazole.

5. The washing or cleaning agent according to any one of claims 1, 3 or 4, **characterised in that** the ligands are ligands of the general formula (III)

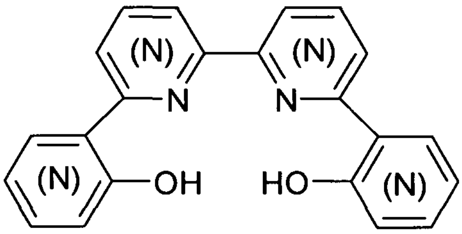

(III)

in which the so formed skeleton can also be bridged as well as mono- or polysubstituted.

6. The washing or cleaning agent according to claim 5, **characterised in that** the ligands are ligands of the general formula (IV)

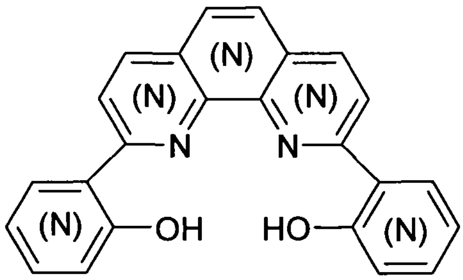

(IV),

in which the skeleton can also be mono- or polysubstituted.

7. The washing or cleaning agent according to claim 6, **characterised in that** the ligands are ligands of the general formula (V)

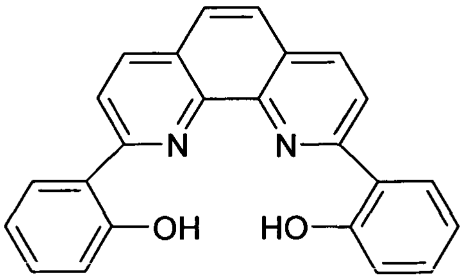

(V)

in which the skeleton can also be mono- or polysubstituted.

8. The washing or cleaning agent according to any one of claims 1 to 7, **characterised in that** the skeleton of the ligand bears as substituent at least one residue comprising a group selected from ammonium, hydroxycarbonyl, alkoxycarbonyl, aryloxycarbonyl, amidocarbonyl, halogen, nitro, sulfato, sulfo, amidosulfo, phosphato, phosphono, amidophosphono, hydroxy, alkoxy, amino and polyoxyethylene.

9. The washing or cleaning agent according to any one of claims 1 to 8, **characterised in that** the metal of the metal-ligand complex is selected from Ag, Al, Ce, Co, Cu, Fe, Mo, Mn, Ni, Pb, Re, Ti, V and Zn in any oxidation states.

10. Use of washing or cleaning agents according to any one of claims 1 to 9 for cleaning textile fabrics.

11. Use of washing or cleaning agents according to any one of claims 1 to 9 for cleaning hard surfaces.

12. Use of ligands and/or ligand-metal complexes of ligands of the general formula (I)

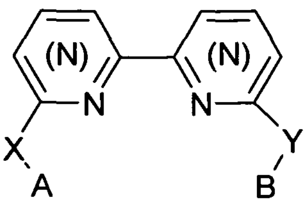
(I),

in which
X and Y mutually independently denote ethyl, ethenyl, -CH=N-, -C($C_{1-18}$-alkyl)=N-, cycloalkyl, cycloheteroalkyl, $C_{6-10}$ aryl or heteroaryl, A and B mutually independently denote OH, SH, $NH_2$, NH($C_{1-18}$-alkyl) or -N($C_{1-18}$-alkyl)$_2$,
in which A is linked by X and B by Y to the biheteroaryl residue via 2 atoms,
in which (N) means that optionally one or two CH groups of the corresponding aryl residue may be replaced by N, and in which the so formed skeleton can also be bridged as well as mono- or polysubstituted,
for cleaning textile fabrics.

**13.** Use of ligands and/or ligand-metal complexes of ligands of the general formula (I)

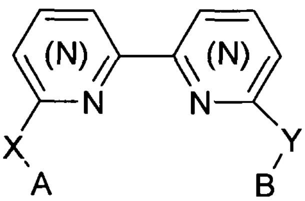
(I),

in which
X and Y mutually independently denote ethyl, ethenyl, -CH=N-, -C($C_{1-18}$-alkyl)=N-, cycloalkyl, cycloheteroalkyl, $C_{6-10}$ aryl or heteroaryl, A and B mutually independently denote OH, SH, $NH_2$, NH($C_{1-18}$-alkyl) or -N($C_{1-18}$-alkyl)$_2$,
in which A is linked by X and B by Y to the biheteroaryl residue via 2 atoms,
in which (N) means that optionally one or two CH groups of the corresponding aryl residue may be replaced by N, and in which the so formed skeleton can also be bridged as well as mono- or polysubstituted,
for cleaning hard surfaces.

**14.** Use of ligands and/or ligand-metal complexes of ligands of the general formula (I)

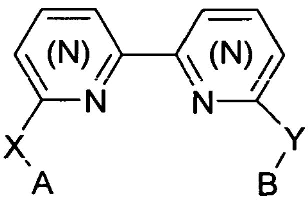
(I),

in which
X and Y mutually independently denote ethyl, ethenyl, -CH=N-, -C($C_{1-18}$-alkyl)=N-, cycloalkyl, cycloheteroalkyl, $C_{6-10}$ aryl or heteroaryl, A and B mutually independently denote OH, SH, $NH_2$, NH($C_{1-18}$-alkyl) or -N($C_{1-18}$-alkyl)$_2$,
in which A is linked by X and B by Y to the biheteroaryl residue via 2 atoms,
in which (N) means that optionally one or two CH groups of the corresponding aryl residue may be replaced by N, and in which the so formed skeleton can also be bridged as well as mono- or polysubstituted,
for bleaching woodpulp and/or raw cotton.

## Revendications

1. Agent de lavage ou de nettoyage contenant des ligands ou des complexes métal-ligand de ligands répondant à la formule générale (I)

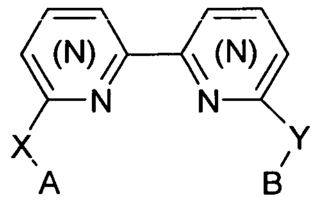

(I),

dans laquelle
X et Y représentent, indépendamment l'un de l'autre, un groupe éthyle, un groupe éthényle, un groupe -CH=N-, un groupe -C(alkyl en $C_1$-$C_{18}$)=N-, un groupe cycloalkyle, un groupe cyclohétéroalkyle, un groupe aryle en $C_6$-$C_{10}$ ou un groupe hétéroaryle ;
A et B représentent, indépendamment l'un de l'autre, un groupe OH, un groupe SH, un groupe $NH_2$, un groupe NH (alkyl en $C_1$-$C_{18}$) ou un groupe N(alkyl en $C_1$-$C_{18}$)$_2$,
A pouvant être relié par X et B par Y via 2 atomes au résidu bihétéroaryle,
la représentation (N) signifiant que, de manière facultative, un ou deux groupes CH du résidu aryle correspondant peuvent être remplacés par N;
et dans lequel le squelette ainsi obtenu peut également être ponté et peut être une ou plusieurs fois substitué.

2. Agent de lavage ou de nettoyage selon la revendication 1, **caractérisé en ce que**, en ce qui concerne les ligands, il s'agit de ligands répondant à la formule générale (II) :

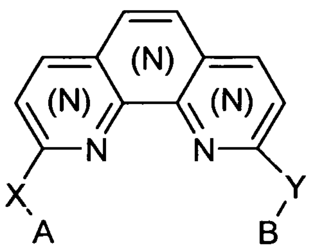

(II)

le squelette ainsi formé pouvant également être une ou plusieurs fois substitué.

3. Agent de lavage ou de nettoyage selon la revendication 1 ou 2, **caractérisé en ce que** X et Y représentent, indépendamment l'un de l'autre, un groupe aryle en $C_6$-$C_{10}$ ou un groupe hétéroaryle ; et A et B représentent un groupe OH ou un groupe SH.

4. Agent de lavage ou de nettoyage selon l'une quelconque des revendications 1 à 3, **caractérisé en ce que** X et Y sont choisis parmi un groupe benzène, un groupe naphtalène, un groupe pyrrol, un groupe furanne, un groupe thiophène, un groupe pyridine, un groupe pyrimidine, un groupe pyrazine, un groupe pyrone, un groupe pyridone, un groupe purine, un groupe triazine, un groupe imidazole, un groupe thiazole, un groupe oxazole, un groupe indole, un groupe quinoléine, un groupe isoquinoléine, un groupe benzimidazole, un groupe benzothiazole ou un groupe benzoxazole, le cas échéant substitué.

5. Agent de lavage ou de nettoyage selon l'une quelconque des revendications 1, 3 ou 4, **caractérisé en ce que**, en ce qui concerne les ligands, il s'agit de ligands répondant à la formule générale (III) :

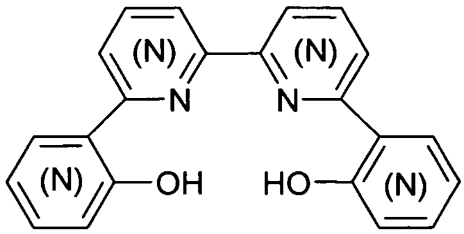

(III)

le squelette ainsi formé pouvant également être ponté et pouvant être une ou plusieurs fois substitué.

**6.** Agent de lavage ou de nettoyage selon la revendication 5, **caractérisé en ce que**, en ce qui concerne les ligands, il s'agit de ligands répondant à la formule générale (IV) :

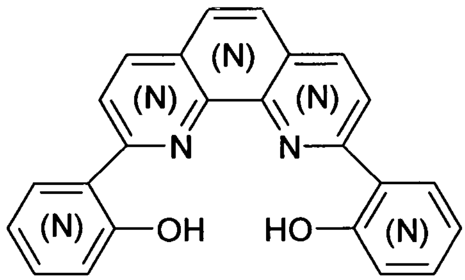

(IV),

le squelette ainsi formé pouvant également être une ou plusieurs fois substitué.

**7.** Agent de lavage ou de nettoyage selon la revendication 6, **caractérisé en ce que**, en ce qui concerne les ligands, il s'agit de ligands répondant à la formule générale (V) :

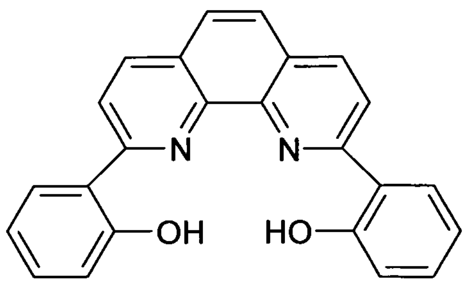

(V)

le squelette ainsi formé pouvant également être une ou plusieurs fois substitué.

**8.** Agent de lavage ou de nettoyage selon l'une quelconque des revendications 1 à 7, **caractérisé en ce que** le squelette du ligand porte au moins un résidu comprenant un groupe choisi parmi un groupe ammonium, un groupe hydroxycarbonyle, un groupe alcoxycarbonyle, un groupe aryloxycarbonyle, un groupe amidocarbonyle, un atome d'halogène, un groupe nitro, un groupe sulfato, un groupe sulfo, un groupe amidosulfo, un groupe phosphato, un groupe phosphono, un groupe amidophosphono, un groupe hydroxyle, un groupe alcoxy, un groupe amino et un groupe polyoxyéthylène, à titre de substituants.

**9.** Agent de lavage ou de nettoyage selon l'une quelconque des revendications 1 à 8, **caractérisé en ce que** le métal du complexe métal-ligand est choisi parmi Ag, Ai, Ce, Co, Cu, Fe, Mo, Mn, Ni, Pb, Re, Ti, V et Zn dans n'importe quels degrés d'oxydation.

**10.** Utilisation d'agents de lavage et de nettoyage selon l'une quelconque des revendications 1 à 9, pour le nettoyage de produits plats textiles.

**11.** Utilisation d'agents de lavage et de nettoyage selon l'une quelconque des revendications 1 à 9, pour le nettoyage de surfaces dures.

**12.** Utilisation de ligands et/ou de complexes métal-ligand de ligands répondant à la formule générale (I)

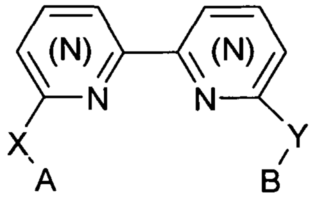

(I),

dans laquelle
X et Y représentent, indépendamment l'un de l'autre, un groupe éthyle, un groupe éthényle, un groupe -CH=N-, un groupe -C(alkyl en $C_1$-$C_{18}$)=N-, un groupe cycloalkyle, un groupe cyclohétéroalkyle, un groupe aryle en $C_6$-$C_{10}$ ou un groupe hétéroaryle ;
A et B représentent, indépendamment l'un de l'autre, un groupe OH, un groupe SH, un groupe $NH_2$, un groupe NH (alkyl en $C_1$-$C_{18}$) ou un groupe N(alkyl en $C_1$-$C_{18}$)$_2$,
A pouvant être relié par X et B par Y via 2 atomes au résidu bihétéroaryle,
la représentation (N) signifiant que, de manière facultative, un ou deux groupes CH du résidu aryle correspondant peuvent être remplacés par N;
et dans lequel le squelette ainsi obtenu peut également être ponté et peut être une ou plusieurs fois substitué, pour le nettoyage de produits plats textiles.

**13.** Utilisation de ligands et/ou de complexes métal-ligand de ligands répondant à la formule générale (I)

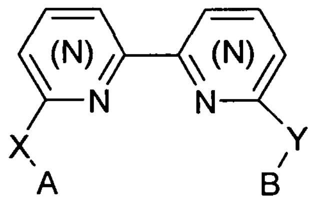

(I),

dans laquelle
X et Y représentent, indépendamment l'un de l'autre, un groupe éthyle, un groupe éthényle, un groupe -CH=N-, un groupe -C(alkyl en $C_1$-$C_{18}$)=N-, un groupe cycloalkyle, un groupe cyclohétéroalkyle, un groupe aryle en $C_6$-$C_{10}$ ou un groupe hétéroaryle ;
A et B représentent, indépendamment l'un de l'autre, un groupe OH, un groupe SH, un groupe $NH_2$, un groupe NH (alkyl en $C_1$-$C_{18}$) ou un groupe N(alkyl en $C_1$-$C_{18}$)$_2$,
A pouvant être relié par X et B par Y via 2 atomes au résidu bihétéroaryle,
la représentation (N) signifiant que, de manière facultative, un ou deux groupes CH du résidu aryle correspondant peuvent être remplacés par N;
et dans lequel le squelette ainsi obtenu peut également être ponté et peut être une ou plusieurs fois substitué, pour le nettoyage de surfaces dures.

**14.** Utilisation de ligands et/ou de complexes ligand-métal de ligands répondant à la formule générale (I)

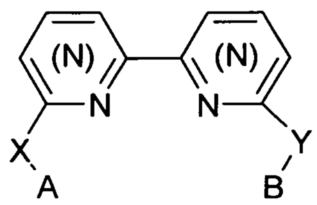

(I),

dans laquelle
X et Y représentent, indépendamment l'un de l'autre, un groupe éthyle, un groupe éthényle, un groupe -CH=N-, un groupe -C(alkyl en $C_1$-$C_{18}$)=N-, un groupe cycloalkyle, un groupe cyclohétéroalkyle, un groupe aryle en $C_6$-$C_{10}$ ou un groupe hétéroaryle ;
A et B représentent, indépendamment l'un de l'autre, un groupe OH, un groupe SH, un groupe $NH_2$, un groupe NH (alkyl en $C_1$-$C_{18}$) ou un groupe N(alkyl en $C_1$-$C_{18}$)$_2$,
A pouvant être relié par X et B par Y via 2 atomes au résidu bihétéroaryle,
la représentation (N) signifiant que, de manière facultative, un ou deux groupes CH du résidu aryle correspondant peuvent être remplacés par N;
et dans lequel le squelette ainsi obtenu peut également être ponté et peut être une ou plusieurs fois substitué, pour le blanchiment de cellulose et/ou de coton brut.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0009512 A
- US 20030205707 A
- WO 0458955 A
- DE 102005053529
- WO 9604940 A


### In der Beschreibung aufgeführte Nicht-Patentliteratur


- **GIBLIN et al.** *Bioorganic & Medicinal Chemistry Letters,* 2001, vol. 11, 1367-1370
- **LIN et al.** *Chem. Eur. J.,* 2003, vol. 9, 1263-1272
- **COUCHMA et al.** *Polyhedron,* 1999, vol. 18, 2633-2640
- **GEISSMAN et al.** *J. Org. Chem.,* 1946, vol. 11, 741-750
- **KONING et al.** *ARKIVOC,* 2004, ISSN 1424-6376, 189-205
- **LAM et al.** *Tetrahedron,* 1999, vol. 55, 8377-8384
- **ROUTIER et al.** *J. Chem. Soc., Perkin Trans.,* 1998, vol. 2, 863-868
- **S. ROUTIER ; V. JOANNY ; A. ZAPARUCHA ; H. VEZIN ; J.-P. CATTEAU ; J.-L. BERNIER ; C. BAILLY.** *J. Chem. Soc., Perkin Trans.,* 1998, vol. 2, 863-868